(19) **Europäisches Patentamt European Patent Office Office européen des brevets**

(11) **EP 4 249 594 A1**

(12) **EUROPEAN PATENT APPLICATION**
published in accordance with Art. 153(4) EPC

(43) Date of publication:
**27.09.2023 Bulletin 2023/39**

(21) Application number: **21894919.6**

(22) Date of filing: **25.10.2021**

(51) International Patent Classification (IPC):
$C12N\ 9/64^{(2006.01)}$   $C12N\ 15/85^{(2006.01)}$
$A61K\ 38/48^{(2006.01)}$   $A61P\ 7/02^{(2006.01)}$
$C07K\ 16/40^{(2006.01)}$   $G01N\ 33/573^{(2006.01)}$
$G01N\ 33/68^{(2006.01)}$

(52) Cooperative Patent Classification (CPC):
**A61K 38/48; A61P 7/02; C07K 16/40; C12N 9/64; C12N 15/85; G01N 33/573; G01N 33/68**

(86) International application number:
**PCT/KR2021/014991**

(87) International publication number:
**WO 2022/108148 (27.05.2022 Gazette 2022/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.11.2020 KR 20200154710**

(71) Applicant: **Green Cross Corporation Yongin-si, Gyeonggi-do 16924 (KR)**

(72) Inventors:
  • **NAM, Hyun-Ja**
    **Yongin-si Gyeonggi-do 16924 (KR)**
  • **HWANG, Sung Ho**
    **Yongin-si Gyeonggi-do 16924 (KR)**
  • **KWAK, Heechun**
    **Yongin-si Gyeonggi-do 16924 (KR)**
  • **CHOI, Gahee**
    **Yongin-si Gyeonggi-do 16924 (KR)**

  • **KIM, Suyong**
    **Yongin-si Gyeonggi-do 16924 (KR)**
  • **KIM, Yu Young**
    **Yongin-si Gyeonggi-do 16924 (KR)**
  • **LEE, Yongmin**
    **Yongin-si Gyeonggi-do 16924 (KR)**
  • **LEE, Chae Mok**
    **Yongin-si Gyeonggi-do 16924 (KR)**
  • **SHIN, Sunhye**
    **Yongin-si Gyeonggi-do 16924 (KR)**
  • **KWON, Young Eun**
    **Yongin-si Gyeonggi-do 16924 (KR)**
  • **JO, Seunghyun**
    **Yongin-si Gyeonggi-do 16924 (KR)**

(74) Representative: **HGF**
  **HGF Limited**
  **1 City Walk**
  **Leeds LS11 9DX (GB)**

Remarks:
The complete document including Reference Table(s) and the Sequence Listing(s) can be downloaded from the EPO website

(54) **ADAMTS13 VARIANT HAVING INCREASED ESCAPING RATE OR ACTIVITY AGAINST AUTOANTIBODY**

(57) The present invention relates to an ADAMTS 13 mutant protein having an improved escaping rate against an autoantibody and a composition for preventing or treating thrombotic diseases using same. By efficiently avoiding representative autoantibodies known to have high binding affinity to the main domain of ADAMTS 13, the ADAMTS 13 variant protein of the present invention can be used as an effective therapeutic composition for various thrombotic diseases, such as TTP (thrombotic thrombocytopenic purpura), etc., in which the presence of such autoantibodies is the main etiology, and can stably maintain the biological activity thereof when administered into a body. In addition, as a new site recognized by an autoantibody is identified within ADAMTS 13, the present invention can be used usefully in screening novel ADAMTS 13 variants having an improved autoantibody escaping rate by applying a combination of various mutations within the corresponding site.

[FIG. 3b]

## Description

### Technical Field

The present invention relates to an ADAMTS 13 variant having reduced or high enzymatic activity for an autoantibody and a composition for treating thrombotic diseases using the same.

### Background Art

Thrombotic thrombocytopenic purpura (TTP) is a rare blood disease belonging to thrombotic microangiopathy, in which blood clots form in small blood vessels throughout the body, leading to death if not treated immediately. The incidence is known to be 1.5 to 6 cases per one million people per year, and the incidence is high mainly in adults with an average age of 40 and women (Miesbach *et al.*, 2019). Its pathological characteristics include a decrease in platelets, a decrease in red blood cells, an increase in hematocrit (HCT), *etc.,* and due to blood clots, dysfunction in many organs, such as the kidneys, heart, brain, is known to occur. Its symptoms include bruising, fever, malaise, dyspnea, confusion of consciousness, headache, *etc.* (Hovinga *et al.*, 2017).

Thrombotic thrombocytopenic purpura (TTP) is divided into two kinds: congenital TTP (cTTP), which causes congenital ADAMTS 13 function deficiency due to dysfunction of the gene encoding ADAMTS 13 (a disintegrin and metalloproteinase with a thrombospondin type 1 motif, member 13); and acquired TTP (aTTP), which is caused by reduced ADAMTS 13 activity due to acquired causes. In general, when ADAMTS 13 activity is less than 10% of normal TTP, it is diagnosed with TTP. It has been reported that aTTP is caused by bacterial infection, certain drugs, autoimmune diseases such as lupus, pregnancy, *etc.,* and as one of the main mechanisms of pathogenesis, inhibition of ADAMTS 13 activity due to autoantibodies recognizing ADAMTS13 has been reported (GARD program, 2018). The ADAMTS13 enzyme plays a role in decomposing large multimers of von Willebrand factor (vWF) into smaller units, in which antibodies found in aTTP patients bind to ADAMTS 13 and inhibit its function, and prevents the degradation of vWF, and this ultimately results in overproduction of blood clots along with platelets thereby causing a disease.

As a standard treatment for cTTP patients, supplemental therapy is used in which a deficient decomposing enzyme is provided through the injection of fresh frozen plasma, whereas for aTTP patients, it is aimed to alleviate symptoms by removing neutralizing antibodies by performing plasma exchange (PEX). In the case of aTTP patients, immunosuppressants (prednisolone, corticosteroid, *etc.*) are administered in combination to increase the treatment effect, prevent recurrence, and rituximab, which induces B cell death, may be used to reduce the production of neutralizing antibodies. Although the number of plasma exchanges depends on the severity of the disease and the progression of symptoms, several plasma exchanges are generally required to normalize the platelet count. Current treatments have limitations such as the inconvenience of repeated plasma exchanges and increased vulnerability to infections resulting from the use of immunosuppressants.

Accordingly, in order to overcome the limitations of conventional therapies for TTP, the present inventors prepared a recombinant ADAMTS 13 protein variant capable of maintaining the activity even in the presence of an autoantibody by efficiently escaping the binding to the patient's autoantibodies, and it was confirmed that the recombinant ADAMTS 13 protein variant can be usefully used as an effective treatment for TTP disease through efficacy evaluation in a TTP disease mouse model.

A number of journal articles and patent documents are referenced throughout the present specification and their citations are indicated. The disclosures of the cited journal articles and patent documents are incorporated herein by reference in their entirety to more clearly describe the level of the technical field to which the present invention pertains and the details of the present invention.

### Disclosure of Invention

### Technical Problem

The present inventors have made an extensive effort to develop an effective and fundamental method for treating various ADAMTS 13 dysfunctional diseases, most of which belong to intractable rare diseases. As a result, they have discovered that when the core regions of ADAMTS 13 recognized by autoantibodies are identified and some amino acids in these regions are substituted, the binding to the autoantibodies is blocked and the activities for vWF degradation and thrombosis inhibition are maintained, and thus can be used as an efficient therapeutic composition for various diseases caused by excessive thrombus formation including thrombotic thrombocytopenic purpura (TTP), thereby completing the present invention.

Accordingly, an object of the present invention is to provide an ADAMTS 13 variant protein or a functional fragment thereof.

Another object of the present invention is to provide a composition for preventing or treating thrombotic disease.

Still another object of the present invention is to provide a method for screening ADAMTS 13 variant proteins with an improved escaping rate for ADAMTS 13 autoantibodies.

Other objects and advantages of the present invention will become more apparent from the following detailed description of the invention, claims and drawings.

**Solution to Problem**

According to an aspect of the present invention, there is provided an ADAMTS 13 (a disintegrin and metalloproteinase with a thrombospondin type 1 motif, member 13) variant protein including substitutions of one or more amino acid residues selected from the group consisting of the 85th, 93rd, 126th, 135th, 278th, 282nd, 308th, 314th, 317th, 334th, 364th, 376th, 413rd, 427th, 452nd, 465th, 567th, 578th, 585th, 589th, 607th, 608th, 609th, 612th, 618th, 624th, 630th, 635th, 643rd, 650th, 651st, 654th, 655th, 656th, 658th, 664th, and 672nd residues of SEQ ID NO: 1, or a functional fragment thereof.

The present inventors have made an extensive effort to develop an effective and fundamental method for treating various ADAMTS 13 dysfunctional diseases, most of which belong to intractable rare diseases. As a result, they have discovered that when the core regions recognized by the ADAMTS13 autoantibody are identified and some amino acids in these regions are substituted, the binding to the autoantibody is blocked and the activities for vWF degradation and thrombosis inhibition are maintained, and thus can be used as an efficient therapeutic composition for various diseases caused by excessive thrombus formation including thrombotic thrombocytopenic purpura (TTP).

As used herein, the term "protein" refers to a linear molecule formed by the mutual binding of amino acid residues by peptide bonds.

As used herein, the term "a functional fragment" refers to an analog of a full-length protein, which, being a fragment of a full-length protein where some amino acid residues have been deleted, maintains its original biological activity and function.

According to the present invention, SEQ ID NO: 1 is the amino acid sequence of the ADAMTS 13 protein consisting of 1427 amino acids. Accordingly, the ADAMTS13 variant protein or a functional fragment thereof of the present invention may be a full-length (1427 a.a.) ADAMTS13 protein or an ADAMTS13 variant in which the above-listed variations are introduced into the functional fragment thereof including the 75th to the 685th region. Some fragments including the 75th to the 685th region may be, for example, 1-685 (685 a.a.) or 75-685 (611 a.a.).

SEQ ID NO: 1, which is an amino acid sequence essentially included in the ADAMTS13 protein and a functional fragment thereof of the present invention, also includes an amino acid sequence showing substantial identity to the sequence above. Substantial identity refers to an amino acid sequence which shows homology of at least 70%, specifically homology of at least 80%, more specifically homology of at least 90%, and most specifically homology of at least 95% to the amino acid sequence above when analyzed using an algorithm commonly used in the art after aligning it with the amino acid sequence above as much as possible.

As used herein, the term "autoantibody" refers to an antibody, which, being one of immunoglobulin proteins including one or more variable domains that bind to an epitope of an antigen thereby capable of specifically recognizing the antigen, is produced by an individual's own immune system, recognizes and targets its own protein. The presence of an autoantibody causes a decrease or loss of an endogenous function or biological activity of a protein specifically recognized by the corresponding autoantibody, and thus becomes the cause of various diseases.

According to a specific embodiment of the present invention, the ADAMTS13 variant protein is selected from the group consisting of each variant protein including substitutions of amino acid residues at the following positions:

- the 85th and 317th residues; the 612th residue; two or more among the 282nd, 465th, and 672nd residues; the 635th residue; the 452nd and 612th residues; two or more among the 278th, 334th, and 427th residues; the 618th residue; the 135th residue; two or more among the 126th, 567th, and 651st residues; the 413th residue; the 334th residue; the 314th residue; two or more among the 93rd, 364th, and 376th residues; the 308th residue; the 656th residue; the 607th residue; the 612 and 624th residues; the 589th residue; the 650th and 656th residues; the 643rd residue; the 585th and 658th residues; two or more among the 630th, 654th, and 664th residues; four or more among the 589th, 608th, 609th, 624th, and 655th residues; the 578th residue; the 585th residue; the 314th and 635th residues; and the 314th and 612th residues.

According to a specific embodiment of the present invention, among the variation positions applied in the present invention, the 85th residue is substituted with Phe, the 93rd residue with Val, the 126th residue with Met, the 135th residue with Ile, the 278th residue with Ile, the 282nd residue with Ala, the 308th residue with Lys, the 314th residue with Thr, the 317th residue with His, the 334th residue with Thr or Val, the 364th residue with Arg, the 376th residue with Asp, the 413th residue with Asp, the 427th residue with Asn, the 452nd residue with Ile, the 465th residue with Asp, the 567th residue with Ser, the 578th residue with Leu, the 585th residue with Asn or Met, the 589th residue with Gln, the 607th

residue with Arg, the 608th residue with Met, the 609th residue with Leu, the612th residue with Phe or Tyr, the 618th residue with Ser, the 624th residue with Asp or Cys, the 630th residue with Leu, the635th residue with Val, the 643rd residue with Phe, the 650th residue with His, the651st residue with Asp, the654th residue with Gly, the 655th residue with Val,the656th residue with Arg or His, the 658th residue with His, the 664th residue with Asn, and the 672nd residue with Val, respectively.

According to another aspect of the present invention, the present invention provides a fusion protein which includes: (a) the above-described ADAMTS 13 variant protein of the present invention or a functional fragment thereof; and (b) an Fc region of the IgG4 immunoglobulin conjugated to (a) above.

The present inventors have found that when an Fc region derived from an IgG4 immunoglobulin is conjugated to the ADAMTS13 variant protein discovered in the present invention, the *in vivo* stability is significantly increased while capable of maintaining the activity of endogenous vWF cleavage and the activity of escaping neutralizing antibodies, and in particular, that structural instability exhibited in open form fragments, in which a part of the distal end has been removed is significantly improved.

According to a specific embodiment of the present invention, the Fc region comprises substitutions of one or more amino acid residues selected from the group consisting of the 22nd, 24th, and 26th residues of SEQ ID NO: 2. More specifically, the 22nd residue is substituted with Tyr, the24th residue with Thr, and the26th residue with Glu, respectively.

According to the present invention, SEQ ID NO: 2 is an Fc region (217 a.a.) derived from an IgG4 immunoglobulin. The present inventors have found that when the ADAMTS 13 variant protein or a functional fragment thereof described above is fused with an IgG4 immunoglobulin-derived Fc region [IgG4 (YTE)] in which the 22nd, 24th, and 26th residues are substituted with Tyr, Thr, and Glu, respectively, the blood half-life of the ADAMTS 13 variant protein or a functional fragment thereof is maximized and thus that the physiological activity after administration can be maintained for a long period of time.

According to a specific embodiment of the present invention, the fusion protein of the present invention further includes a hinge region of an IgG1 immunoglobulin between (a) and (b) described above.

According to the present invention, the hinge region derived from an IgG1 immunoglobulin may be represented by SEQ ID NO: 3 (15 a.a.).

According to still another aspect of the present invention, the present invention provides the above-described ADAMTS 13 variant protein of the present invention or a functional fragment thereof; or a nucleotide encoding the above-described fusion protein.

As used herein, the term "nucleotide" has a meaning comprehensively including DNA (gDNA and cDNA) and RNA molecules, and nucleotides, which are the basic structural units in nucleic acid molecules, include not only natural nucleotides but also analogs thereof in which a sugar or base region is modified. It is apparent to those skilled in the art that the nucleotide sequence to be expressed for the purpose of treatment, drug production, *etc.* in the present invention is not limited to the nucleotide sequence described in the accompanying sequence listing. Some variations in a nucleotide do not cause a change in a protein. Such nucleic acid encompasses all molecules which have a functionally equivalent codon, a codon encoding the same amino acid due to codon degeneracy, and a codon encoding a biologically equivalent amino acid.

Considering the above-described variations having biologically equivalent activity, the nucleotides to be used in the present invention are interpreted as including sequences showing substantial identity to the sequences described in the sequence listing. The substantial identity refers to a sequence which shows homology of at least 70%, homology of at least 80%, more specifically homology of at least 90%, and most specifically homology of at least 95% to the sequence of the present invention when analyzed using an algorithm commonly used in the art after aligning it with the amino acid sequence above as much as possible. The alignment methods for sequence comparison are disclosed in the art. Various methods and algorithms for alignment are described in Huang et al., Comp. Appl. BioSci. 8:155-65 (1992) and Pearson et al., Meth. Mol. Biol. 24:307-31 (1994). The NCBI Basic Local Alignment Search Tool (BLAST) (Altschul et al., J. Mol. Biol. 215:403-10 (1990)) is accessible from the National Center for Biological Information (NCBI), *etc.,* and it can be used in conjunction with sequencing programs such as blastp, blastn, blastx, tblastn, and tblastx.

The nucleotides of the present invention may be used in the form of gene therapy for delivering the above-described variant ADAMTS 13 of the present invention to a subject in the form of a gene, or may be used for the production of a recombinant protein in a pharmaceutical form by expressing the ADAMTS 13 variant protein in a host cell.

As used herein, the term "express" means that in order to allow a subject to express an endogenous gene or introduce the same using a gene carrier so as to increase the natural expression level of an endogenous gene, thereby making the gene replicable as an extrachromosomal factor or by completion of chromosomal integration within a cell of the subject. Therefore, the term "expression" is synonymous with "transformation", "transfection", and "transduction."

As used herein, the term "gene carrier" refers to any means of transporting a gene into a cell, and gene delivery has the same meaning as transduction of a gene in a cell. At the tissue level, the term gene delivery has the same meaning as the spread of a gene. Accordingly, the gene delivery system of the present invention can be described as a gene penetration system and a gene spread system.

The gene delivery system of the present invention may be included in the form of an expression cassette, which is a polynucleotide construct including all elements necessary for self-expression of a gene to be introduced. The expression cassette usually includes a promoter operably linked to the gene, a transcription termination signal, a ribosome binding site, and a translation termination signal. The expression cassette may be in the form of an expression vector capable of self-replication.

The gene delivery system used in the present invention can be applied to any gene delivery system used for conventional gene insertion, and may include, for example, plasmids, adenoviruses, adeno-associated viruses (AAV), retroviruses, and lentiviruses, herpes simplex virus, vaccinia virus, liposomes, and niosomes, but is not limited thereto.

According to still another aspect of the present invention, the present invention provides a composition for preventing or treating thrombotic disease, which includes the above-described ADAMTS 13 variant protein or a functional fragment thereof of the present invention; the above-described fusion protein; or a nucleotide encoding the same as an active ingredient.

According to still another aspect of the present invention, the present invention provides a method for preventing or treating a thrombotic disease, which includes administering to a subject the composition including the above-described ADAMTS13 variant protein or a functional fragment thereof of the present invention; the above-described fusion protein; or a nucleotide encoding the same as an active ingredient.

As used herein, the term "thrombotic disease" refers to a systemic disease in which blood flow is reduced or blocked due to thrombus generated by platelet aggregation in the microcirculatory system of blood vessels, whereby ischemic damage is caused to each organ, such as the kidney, heart, and brain.

When ADAMTS 13 enzyme activity is inhibited by a neutralizing antibody and thus does not properly degrade the von Willerant factor (vWF), excessive platelet aggregation and thrombus overproduction occur. Therefore, the ADAMTS13 variant protein of the present invention, in which the vWF degradation activity are maintained or improved while escaping neutralizing antibodies with high efficiency, can be used as a composition for effective prevention or treatment of various thrombotic diseases.

As used herein, the term "prevention" refers to inhibiting the occurrence of a disorder or disease in a subject who has never been diagnosed with the disorder or disease, but is likely to have the disorder or disease.

As used herein, the term "treatment" refers to (a) inhibiting the progression of a disorder, disease, or symptom; (b) alleviation of the disorder, disease, or symptom; or (c) eliminating the disorder, disease, or symptom. When the composition of the present invention is administered to a subject, it plays the role of specifically recognizing and degrading vWF regardless of the presence of a neutralizing antibody to block the generation of excessive thrombus, thereby inhibiting, eliminating, or alleviating the progression of the thrombotic disease. Accordingly, the composition of the present invention as-is may become a composition for treating these diseases, or may be administered together with other pharmacological ingredients and applied as a therapeutic adjuvant for the above diseases. Accordingly, as used herein, the term "treatment" or "therapeutic agent" includes the meaning of "supplementary treatment" or "therapeutic adjuvant."

As used herein, the term "administration" or "administer" refers to directly administering a therapeutically effective amount of the composition of the present invention to a subject so that the same amount is formed in the subject's body.

As used herein, the term "therapeutically effective amount" refers to the content of the composition in which the pharmacological component contained in the composition is sufficient to provide a therapeutic or prophylactic effect to an individual to whom the pharmaceutical composition of the present invention is to be administered, and it includes the meaning of "prophylactically effective amount."

As used herein, the term "subject" includes, without limitation, a human, mouse, rat, guinea pig, dog, cat, horse, cow, pig, monkey, chimpanzee, baboon, and rhesus monkey. Specifically, the subject of the present invention is a human.

According to a specific embodiment of the present invention, the thrombotic disease that can be prevented or treated by the composition of the present invention is thrombotic microangiopathy (TMA). More specifically, the thrombotic microangiopathy (TMA) is thrombotic thrombocytopenic purpura (TTP), hemolytic uremic syndrome (HUS), HELLP (Hemolysis, Elevated Liver enzymes, and Low Platelet count) syndrome, preeclampsia, and sickle cell disease, more specifically thrombotic thrombocytopenic purpura (TTP) or sickle cell disease, and even more specifically thrombotic thrombocytopenic purpura (TTP).

When the composition of the present invention is prepared as a pharmaceutical composition, the pharmaceutically acceptable carriers to be included therein are those commonly used in formulation, and they include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, *etc.,* but are not limited thereto. The pharmaceutical composition of the present invention may further include a lubricant, a wetting agent, a sweetening agent, a flavoring agent, an emulsifying agent, a suspending agent, a preservative, *etc.,* in addition to the above components. Suitable pharmaceutically acceptable carriers and agents are described in detail in Remington's Pharmaceutical Sciences (19th ed., 1995).

The pharmaceutical composition of the present invention may be administered orally or parenterally, specifically it may be administered in a parenteral manner, and more specifically it may be administered subcutaneously, transdermally, or intravenously.

A suitable dose of the pharmaceutical composition of the present invention may be variously prescribed depending on factors such as a formulation method, administration method, age, weight, sex, health condition, food, administration time, administration route, excretion rate, and reaction sensitivity of the patient. A preferred dose of the pharmaceutical composition of the present invention is in the range of 0.001 mg/kg to 100 mg/kg for adults.

The pharmaceutical composition of the present invention may be prepared in a unit dosage form by formulating using a pharmaceutically acceptable carrier and/or excipient according to a method that can easily be performed by a person of ordinary skill in the art to which the present invention pertains or it may be prepared by incorporation into a multi-dose container. In this case, the formulation may be in the form of a solution in oil or an aqueous medium, suspension, syrup, or emulsion, or may be in the form of an extract, powder, powdered agent, granule, tablet, or capsule, and may additionally include a dispersant or stabilizer.

According to still another aspect of the present invention, the present invention provides a method for screening ADAMTS 13 variants with an improved autoantibody escaping rate, including the steps of:

(a) preparing an ADAMTS 13 variant, in which one or more amino acid residues selected from the group consisting of the 85th, 93rd, 126th, 135th, 278th, 282nd, 308th, 314th, 317th, 334th, 364th, 376th, 413rd, 427th, 452nd, 465th, 567th, 578th, 585th, 589th, 607th, 608th, 609th, 612th, 618th, 624th, 630th, 635th, 643rd, 650th, 651st, 654th, 655th, 656th, 658th, 664th, and 672nd residues of a first sequence of sequence listing are substituted or deleted; and

(b) contacting an autoantibody against ADAMTS 13 with the ADAMTS 13 variant prepared in step (a) above; wherein when the autoantibody binds to the ADAMTS 13 variant with an affinity lower than that for wild-type ADAMTS 13, the ADAMTS 13 variant is determined as an ADAMTS 13 variant with an improved escaping rate for autoantibodies.

According to the present invention, the present inventors have analyzed a library of variants of about 800 or so obtained by random mutagenesis as to whether or not they could bind to the ADAMTS 13 neutralizing antibody using; as a result, they have found that the positions of the amino acid residues in SEQ ID NO: 1 play an important role in binding to the neutralizing antibody, and that the affinity for the neutralizing antibody can be controlled by variations at these positions. Accordingly, various variants can be derived through the type of variation and a combination of variations thereof introduced through these residue positions discovered by the present inventors, and ADAMTS 13 variant enzymes whose activities are not reduced by the presence of autoantibodies can be selected through the measurement of the binding of these variants with autoantibodies.

As used herein, the term "autoantibody escaping rate" refers to a value expressed as a percentage of the ratio of ADAMTS 13 variants that do not bind to autoantibodies compared to wild-type ADAMTS 13, and a high escaping rate for autoantibodies means that the affinity for autoantibodies is low and thus the unique biological activity of ADAMTS 13 (e.g., vWF-degrading activity) is not inhibited by the autoantibody. Therefore, the term "ADAMTS 13 variant with an improved autoantibody escaping rate" can also be expressed as "ADAMTS 13 variant with reduced affinity for autoantibodies" or "ADAMTS 13 variant with reduced binding ability to autoantibodies."

According to the present invention, the escaping rate of a candidate variant for autoantibodies can be evaluated by measuring the binding between the ADAMTS 13 variant prepared in step (a) above and autoantibodies. The binding to autoantibodies can be measured by various methods, and one of which is a method which includes culturing antibodies with antigens (or cells expressing the same), removing unbound antibody (e.g., washing), and detecting the bound antibodies with labeled antibodies that bind thereto. The binding between antigens and antibodies is typically mediated through the complementarity determining regions (CDRs) of the antibodies and epitopes of the antigens, and unlike the random and non-specific attachment of proteins, the specific three-dimensional structure of the antigens and the variable domains allows these two structures to form an accurate binding. Therefore, if an appropriate combination of variations in the key reaction site with the autoantibodies discovered through the present invention is made in an appropriate combination, the binding of the autoantibodies can be effectively blocked.

As a result of the measurement, a candidate variant having reduced affinity or binding ability for autoantibodies compared to the wild-type may be determined as an ADAMTS 13 variant having an improved escaping rate for autoantibodies.

As used herein, the term "decrease in affinity" refers to a significant decrease in the binding between an ADAMTS 13 variant and an autoantibody to the extent that the intrinsic enzyme activity of ADAMTS 13 is improved to a measurable level, which can also be described as "reduction of binding ability". Specifically, it refers to a state in which the binding is decreased by 20% or more, more specifically by 40% or more, and more specifically by 60% or more, compared to the wild-type.

According to a specific embodiment of the present invention, step (a) above is performed by substituting one or more amino acid residues selected from the group consisting of the 85th, 93rd, 126th, 135th, 278th, 282nd, 308th, 314th,

317th, 334th, 376th, 413rd, 427th, 465th, 567th, 578th, 585th, 607th, 609th, 612th, 624th, 630th, 643rd, 650th, 654th, 655th, 656th, 658th, and 672nd residues.

## Advantageous Effects of Invention

The characteristics and advantages of the present invention may be summarized as follows:

(a) The present invention provides an ADAMTS13 variant protein with an improved escaping rate for autoantibodies and a composition for preventing or treating thrombotic diseases using the same.

(b) The ADAMTS 13 variant protein of the present invention, by way of efficiently escaping representative autoantibodies that are known to have high binding affinity to the main domain of ADAMTS 13, can be used as an efficient therapeutic composition for various thrombotic diseases, such as thrombotic thrombocytopenic purpura (TTP) in which autoantibodies become the main etiology, and can stably maintain the biological activity when administered into the body.

(c) Additionally, in the present invention, as also a new site recognized by autoantibodies is identified within ADAMTS 13, a combination of various variations can be applied within the corresponding site and thereby can be effectively used for screening novel ADAMTS 13 variants with an improved escaping rate for autoantibodies.

## Brief Description of Drawings

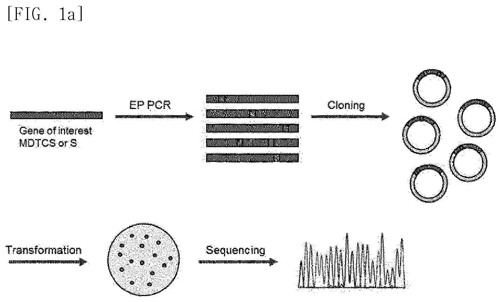

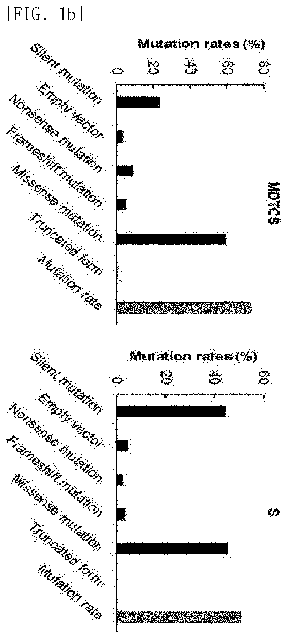

FIG. 1 shows diagrams illustrating the construction of a human ADAMTS 13 variant library using random mutagenesis and the results of confirming the variation rate. FIG. 1a is a schematic diagram of a library construction process by random mutagenesis. Random variations were induced in the MDTCS region or S domain through error-prone PCR (EP PCR), inserted into the acceptor vector, cloned, and then transformed into *E. coli* to secure colonies. DNA was extracted from the colonies and the mutated nucleotide sequences were confirmed through Sanger sequencing analysis. FIG. 1b shows graphs illustrating the results of sequencing analysis. The total variation rate and variation form in the library constructed by targeting the MDTCS or S domain were confirmed, respectively.

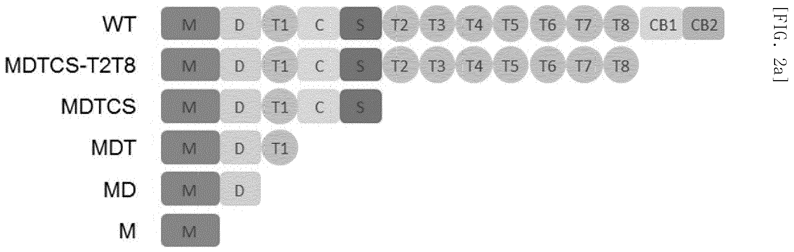

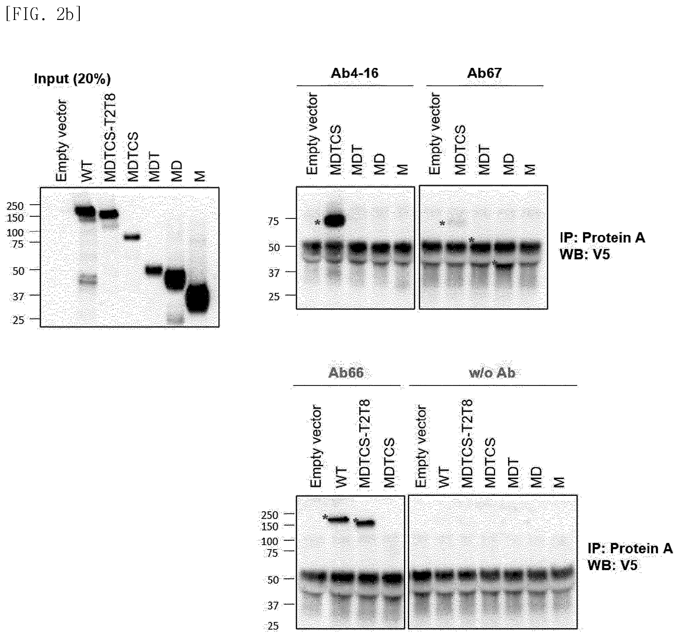

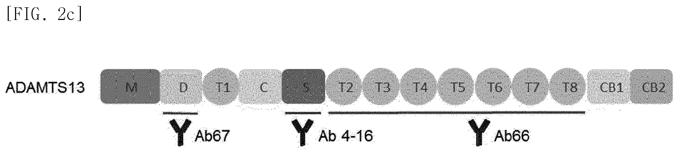

FIG. 2 shows diagrams illustrating the results of confirming the binding site of each domain of ADAMTS 13 that binds to the neutralizing antibody. FIG. 2a shows a schematic diagram of six kinds of ADAMTS 13 fragments (or wild-type full-length ADAMTS 13), in which various domains are combined, prepared to confirm the expression rate of ADAMTS 13 or the binding site of a neutralizing antibody. FIG. 2b shows the results of performing western blotting with an anti-V5 antibody after immunoprecipitation by mixing Protein A-Sepharose and each neutralizing antibody using proteins expressed in each ADAMTS 13 domain. "Input" shows the expression level of the protein for each domain. FIG. 2c is a schematic diagram of the binding site of the ADAMTS13 neutralizing antibody illustrating that Ab 4-16 antibody specifically binds to the S domain, the Ab 67 antibody to the D domain, and the Ab 66 antibody to the T2-T8 domain, respectively.

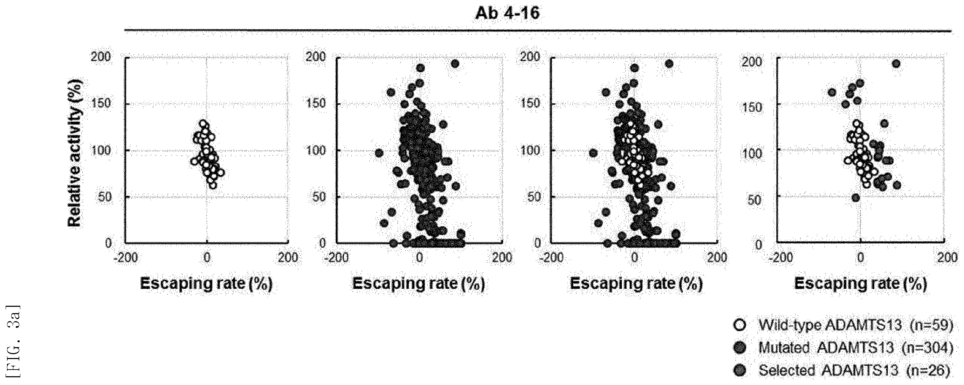

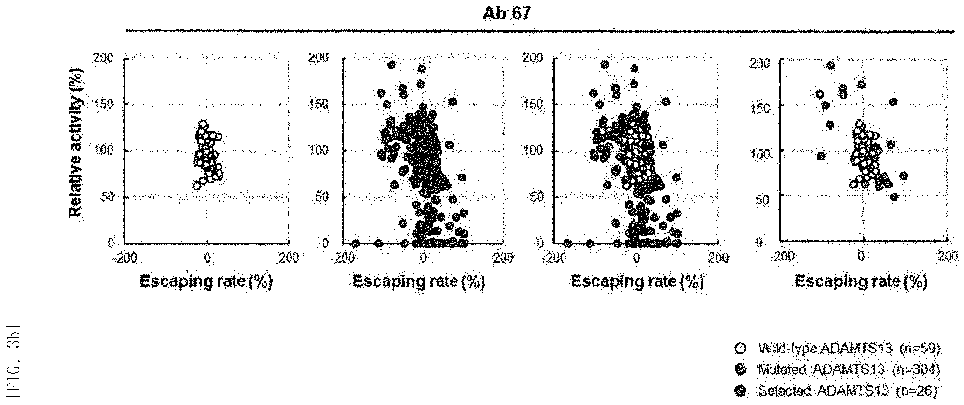

FIG. 3 shows diagrams illustrating the screening results of variants that escape anti-ADAMTS13 antibodies or have superior activity to wild-type ADAMTS 13. The escaping rate and ADAMTS13 activity for Ab 4-16 antibody or Ab 67 antibody were measured in the wild-type clones and the ADAMTS 13 variants. Relative activity was calculated by confirming the specific titers of the wild-type clones and the variants and substituting them in the following equation: Relative activity (%) = specific titer of variants/ specific titer of wild-type ADAMTS 13 × 100.

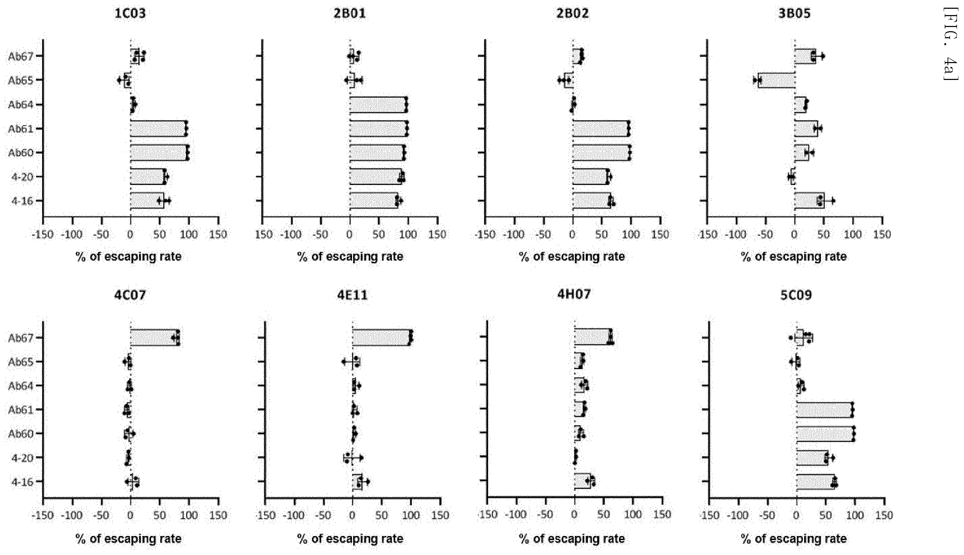

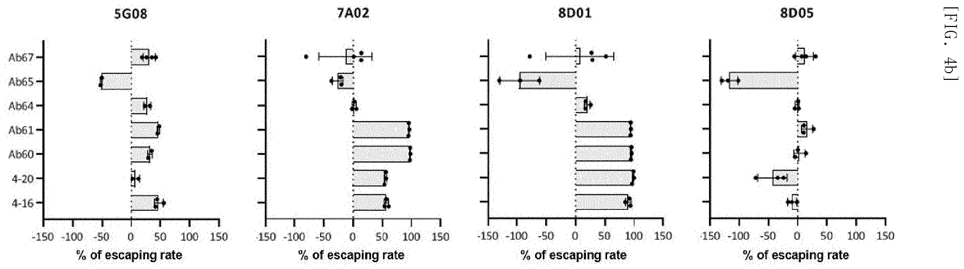

FIG. 4 shows the results confirming the escaping rate of 12 kinds of full-length ADAMTS 13 variants for single neutralizing antibodies. Antibody escaping rates for 7 kinds of neutralizing antibodies (Ab4-16, Ab4-20, Ab60, Ab61, Ab64, Ab65, and Ab67) were measured in the 12 kinds of full-length ADAMTS 13 variants. The neutralizing antibody escaping rate was calculated by comparing the relative binding level with the WT ADAMTS 13 value, and substituting the relative escaping rate into the following equation using the same: Escaping rate (%) = (1 - binding ability of variants/binding ability of WT ADAMTS 13) × 100.

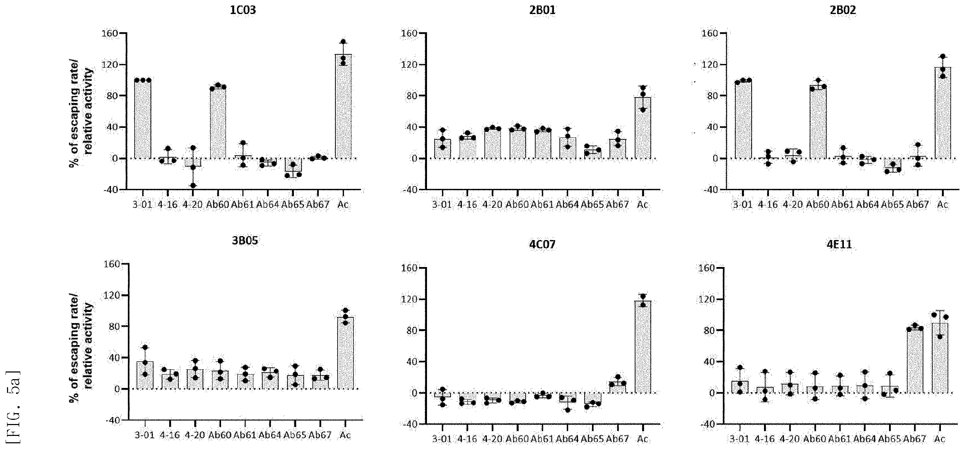

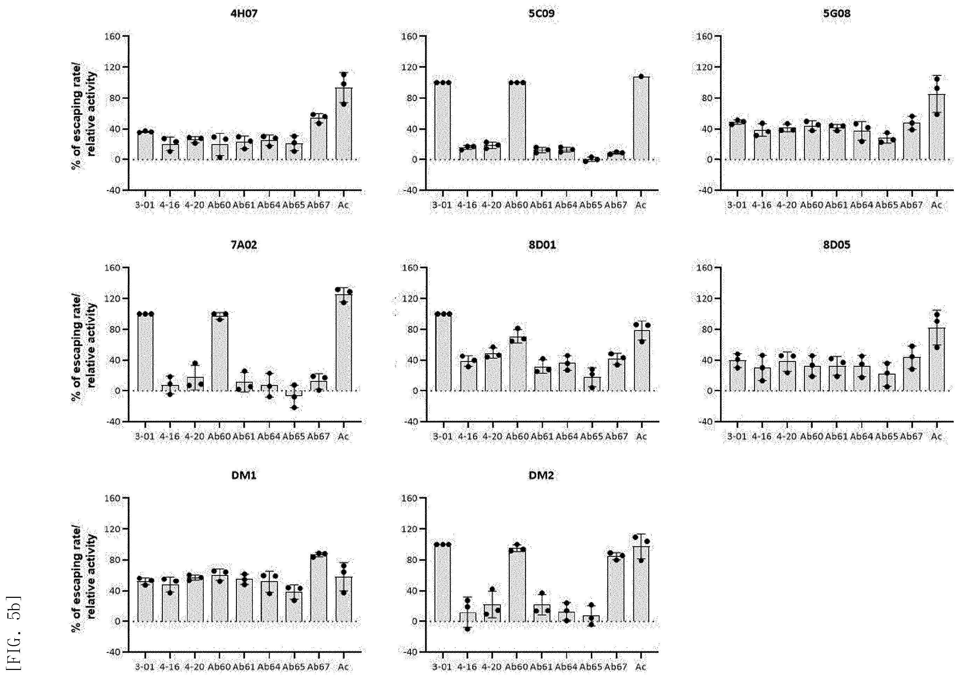

FIG. 5 shows the results confirming the ability to escape single neutralizing antibodies in a culture medium expressing an Fc-attached MDTCS fragment variant. The selected 12 kinds of variants were subjected to MDTCS fragmentation, and then the Fc-attached variants were combined with the selected mutated amino acid residues, and the neutralizing antibody escaping rate and the relative activity of ADAMTS 13 of eight kinds of neutralizing antibodies (Ab3-01, Ab4-16, Ab4-20, Ab60, Ab61, Ab64, Ab65, and Ab67) were measured using a culture medium in which a total of 14 kinds of variants including DM1 and DM2 variants having two amino acid variations were expressed. Escaping rate (%) = (1 - binding ability of variants/binding ability of WT ADAMTS 13) × 100; relative activity (%) = specific titer of variants/specific titer of WT ADAMTS 13 × 100

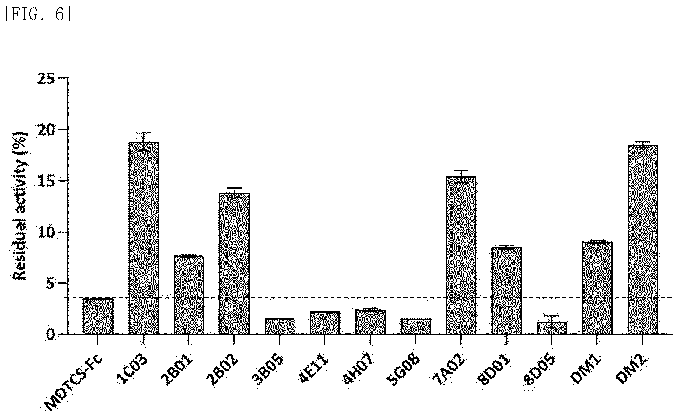

FIG. 6 shows the results confirming the ability of escaping mixed neutralizing antibodies under a condition of a culture medium expressing an Fc-attached MDTCS fragment variant. Nine kinds of neutralizing antibodies (Ab3-01, Ab4-16, Ab4-20, Ab60, Ab61, Ab64, Ab65, Ab66, and Ab67), which were mixed at an equal ratio, were mixed into a 4 nM culture medium expressing MDTCS-Fc (i.e., a control group) and 12 kinds of candidate variants, and allowed to react. Then, the residual activity of each candidate variant was calculated by substituting into the following equation: Residual activity (%) = A ÷ B × 100 (A: activity under the condition of mixed neutralizing antibodies, B: activity under the condition of untreated with neutralizing antibodies).

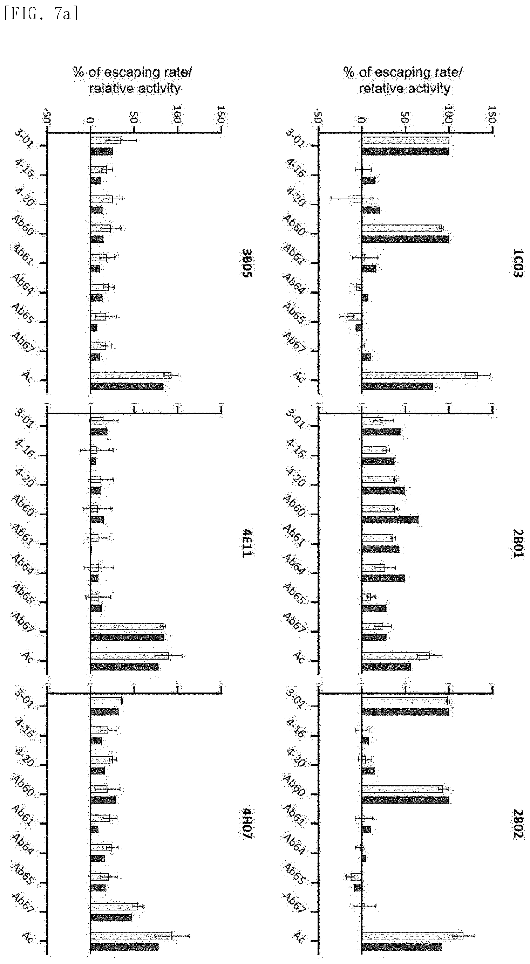

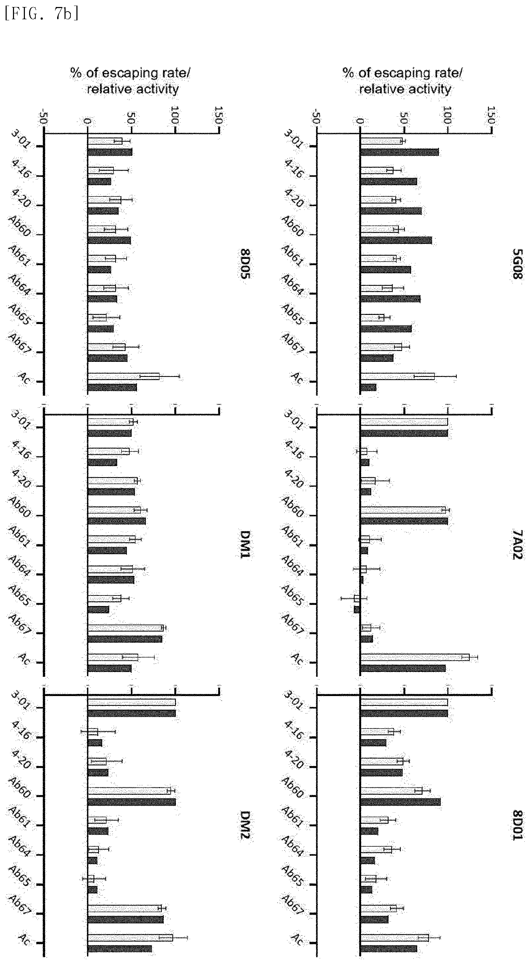

FIG. 7 shows the results confirming the ability of escaping single neutralizing antibodies under the conditions of an Fc-attached MDTCS fragment variant culture medium and a purifying solution. The escaping rate and relative activity of neutralizing antibodies for eight kinds of single neutralizing antibodies (Ab3-01, Ab4-16, Ab4-20, Ab60, Ab61, Ab64, Ab65, and Ab67) were measured using the culture medium in which 12 kinds of variants (1C03, 2B01, 2B02, 3B05, 4E11, 4H07, 5G08, 7A02, 8D01, 8D05, DM1, and DM2) were expressed or a purifying solution purified using the Phytip system (FIGS. 7a and 7b). The concentration of the purifying solution was confirmed through Fc ELISA. Escaping rate (%) = (1- binding ability of variants/binding ability of MDTCS-Fc) × 100, Relative activity (%) = specific titer of variants/specific titer of MDTCS-Fc × 100. Blue bars indicate purified variant proteins and gray bars indicate the median values of the expressed variant proteins, respectively.

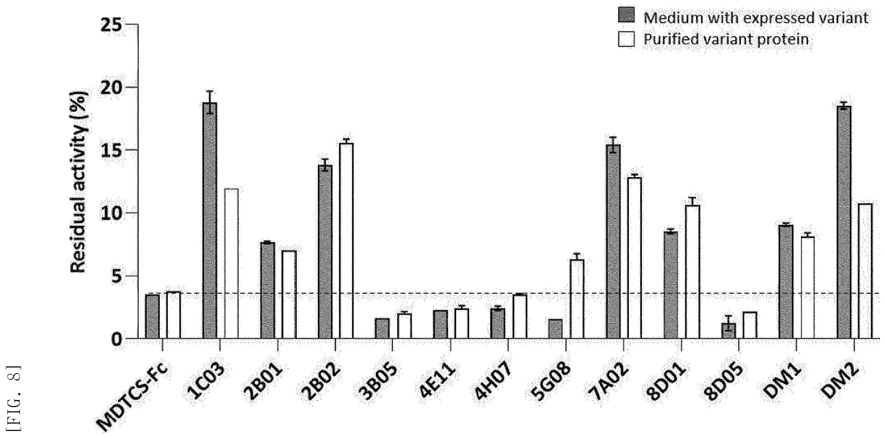

FIG. 8 shows the results confirming the ability of escaping mixed neutralizing antibodies under the conditions of an Fc-attached MDTCS fragment variant culture medium and a purifying solution. Eight kinds of neutralizing antibodies (Ab3-01, Ab4-16, Ab4-20, Ab60, Ab61, Ab64, Ab65, and Ab67), which were mixed at an equal ratio, were mixed into a culture medium expressing MDTCS-Fc (i.e., a control group) and 12 kinds of candidate variants or a 4 nM purifying solution, and allowed to react. Then, the residual activity of each candidate variant was measured. The residual activity was calculated by substituting into the following equation: residual activity (%) = A ÷ B × 100 (A: activity under the condition of mixed neutralizing antibodies, B: activity under the condition of untreated with neutralizing antibodies).

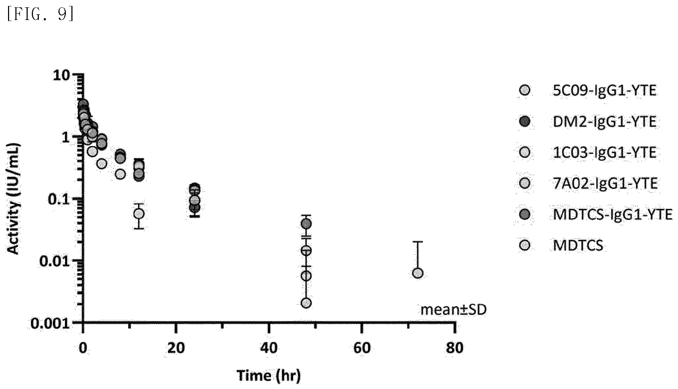

FIG. 9 shows the graphs illustrating the pharmacokinetic results of Fc-attached MDTCS fragment variants. After administration of MDTCS or Fc (IgG1-YTE)-attached MDTCS and the four final candidate variants (1C03, 5C09, 7A02, and DM2) fragment proteins to the tail vein of mice, and plasma was obtained every hour. The protein was administered so that the specific titer of each material can be 160 IU/kg, and the activity of the materials remaining in the plasma obtained every hour was measured through the activity assay.

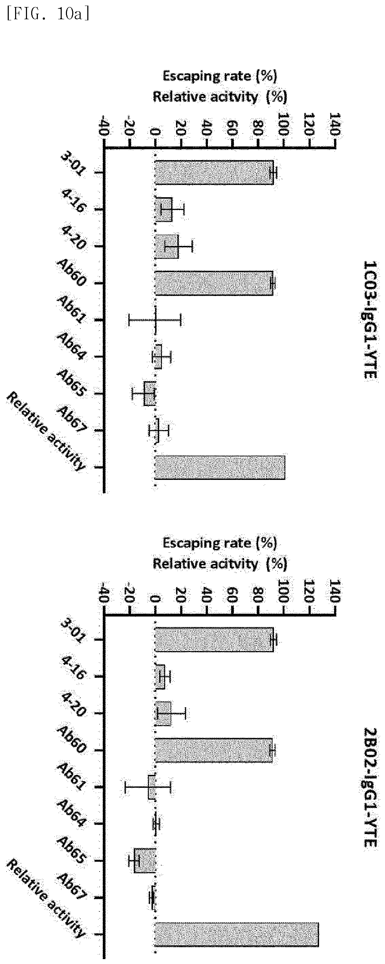

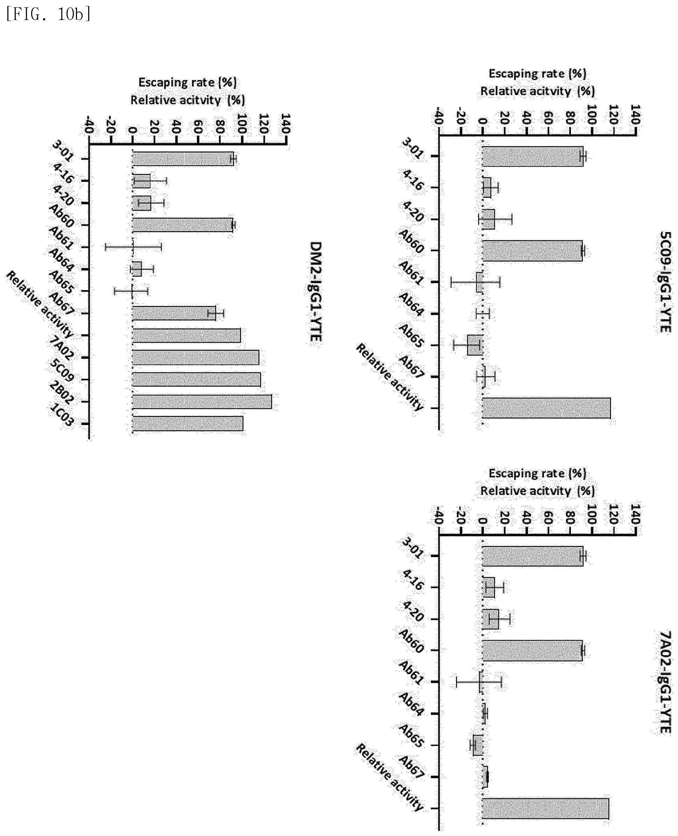

FIG. 10 shows the results confirming the ability of escaping single neutralizing antibodies under the condition of an IgG1-YTE-attached MDTCS fragment variant culture medium. The selected five kinds of variants were subjected to MDTCS fragmentation, and the rate of escaping neutralizing antibodies and relative activity of the eight kinds of single neutralizing antibodies (Ab3-01, Ab4-16, Ab4-20, Ab60, Ab61, Ab64, Ab65, and Ab67) were measured using the culture medium in which the IgG1-YTE-attached variants were expressed (FIGS. 10a and 10b). Escaping rate (%) = (1 - binding ability of variants/binding ability of MDTCS- IgG 1- YTE) × 100; Relative activity (%) = specific titer of variants/specific titer ofMDTCS-IgG1-YTE × 100.

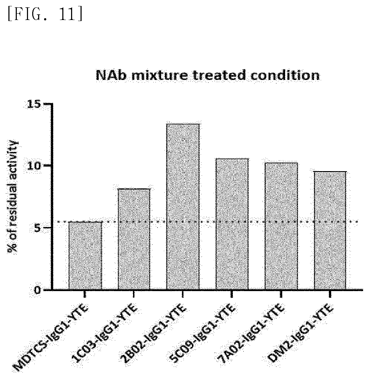

FIG. 11 shows the results confirming the ability of escaping mixed neutralizing antibodies under a condition of a culture medium expressing an IgG1-YTE-attached MDTCS fragment variant. Nine kinds of neutralizing antibodies (Ab3-01, Ab4-16, Ab4-20, Ab60, Ab61, Ab64, Ab65, Ab66, and Ab67), which were mixed at an equal ratio, were mixed into a 4 nM culture medium expressing MDTCS-IgG1-YTE (i.e., a control group) and five kinds of candidate variants, and allowed to react. Then, the residual activity of each candidate variant was calculated by the following equation: Residual activity (%) = A ÷ B × 100 (A: activity under the condition of mixed neutralizing antibodies, B: activity under the condition of untreated with neutralizing antibodies).

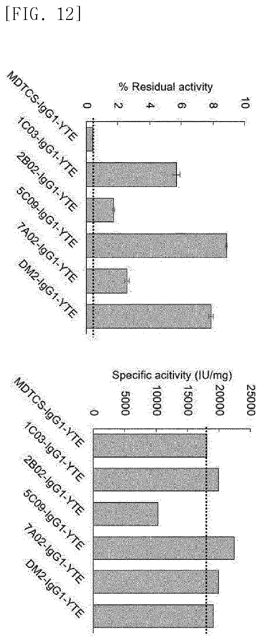

FIG. 12 shows the results confirming the ability of escaping mixed neutralizing antibodies under the condition of an IgG1-YTE-attached MDTCS fragment variant culture medium. Nine kinds of neutralizing antibodies (Ab3-01, Ab4-16, Ab4-20, Ab60, Ab61, Ab64, Ab65, Ab66, and Ab67), which were mixed at an equal ratio, were mixed into a 4 nM purifying solution with MDTCS-IgG1-YTE (i.e., a control group) and five kinds of candidate variants, and allowed to react. Then, the residual activity of each candidate variant was calculated by the following equation: Residual activity (%) = A ÷ B × 100 (A: activity under the condition of mixed neutralizing antibodies, B: activity under the condition of untreated with neutralizing antibodies).

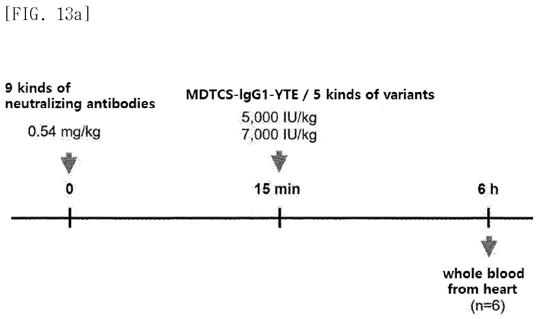

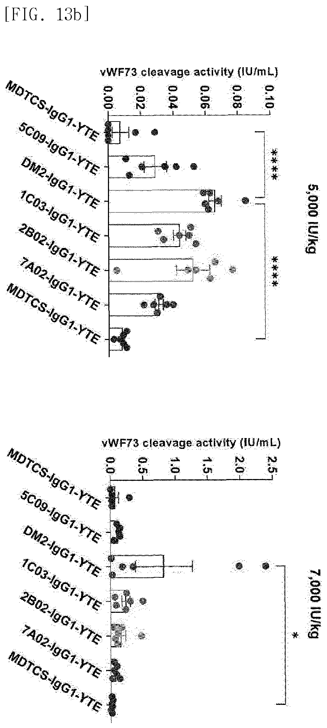

FIG. 13 shows a schematic diagram illustrating a procedure for testing the rate of escaping neutralizing antibodies using an aTTP-mimic mouse model (FIG. 13a) and a diagram illustrating the residual ADAMTS 13 activity according to the dose of variant candidates (FIG. 13b), respectively.

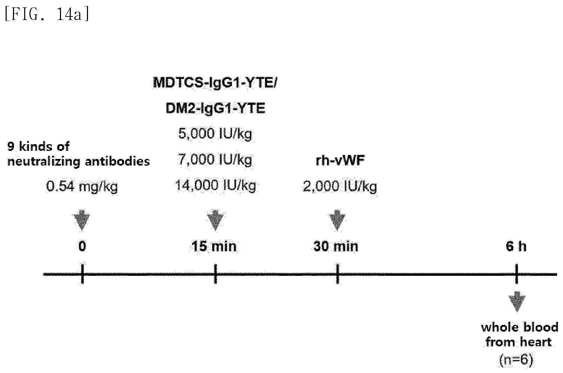

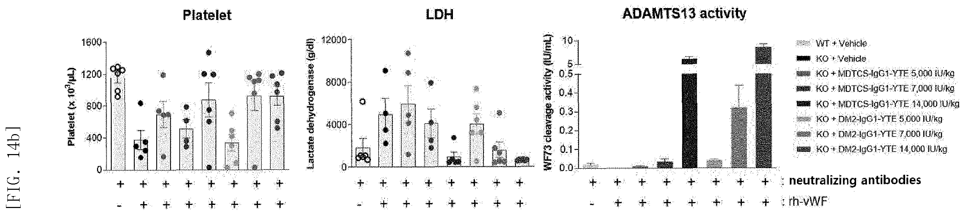

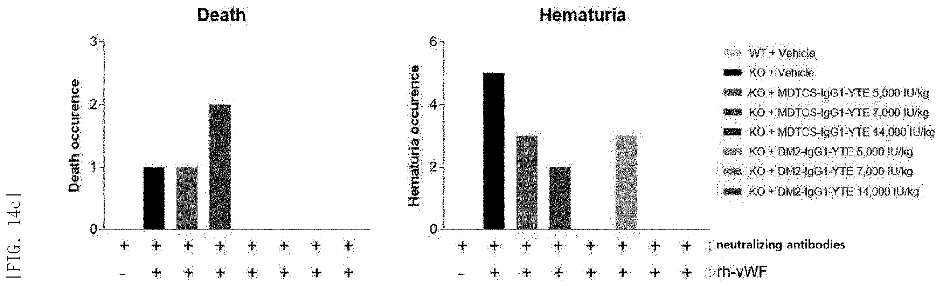

FIG. 14 shows a schematic diagram illustrating a test procedure for maintaining residual activity of ADAMTS 13 and clinical symptoms according to the concentration of DM2-IgG1-YTE, which showed the most excellent neutralizing antibody escaping rate using an aTTP-mimic mouse model (FIG. 14a), a schematic diagram illustrating an improvement in the platelet and LDH levels and ADAMTS 13 activity (FIG. 14b), and the observation results of clinical symptoms (FIG. 14c), respectively.

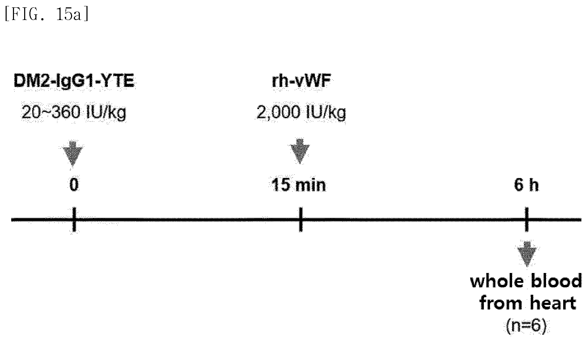

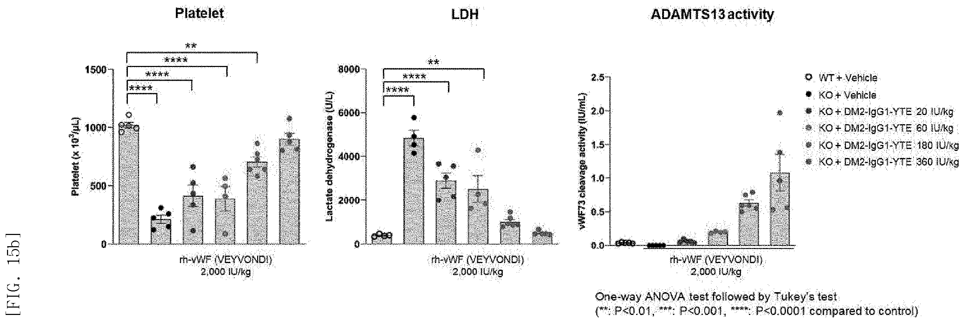

FIG. 15 shows a schematic diagram illustrating a test procedure for the presence/absence of an improvement in hematological and clinical symptoms and the degree of recovery of human ADAMTS 13 activity by administering DM2-IgG1-YTE to a cTTP mouse model (FIG. 15a), and an improvement of platelet and LDH levels and ADAMTS 13 activity, and the observation results of the recovery of ADAMTS 13 activity (FIG. 15b), respectively.

**Mode for the Invention**

Hereinafter, the present invention will be described in more detail through examples. These examples are only for illustrating the present invention in more detail, and it will be apparent to those of ordinary skill in the art that the scope of the present invention is not limited by these examples according to the gist of the present invention.

**Examples**

**Experimental method**

*Construction of human ADAMTS13 variant library through random mutagenesis*

An expression vector containing human wild-type ADAMTS 13 and two receptor vectors for cloning of mutated MDTCS (**M**etalloprotease, **D**isintegrin-like, **T**hrombospondin type 1 (TSP1) repeat, **C**ys-rich, and **S**pacer) or Spacer (S) domain regions were prepared. Error-prone PCR (GeneMorph II Random Mutagenesis Kit, Agilent Technologies) was performed for the MDTCS or S domain region of ADAMTS 13 using the oligonucleotides of Table 1 below, and the PCR product for the amplified MDTCS or S domain was obtained by elution after agarose gel electrophoresis eluted by electrophoresis. After ligation of the eluted PCR product and the acceptor vectors using the Golden Gate cloning method, the resultant was transformed into *Escherichia coli (E. coli)* to prepare a library of variants produced by random mutagenesis. Plasmid DNA of a transfection grade was extracted from 384 E. *coli* colonies per library using the Plasmid Plus 96 Miniprep kit (QIAGEN) to obtain a total of 768 mutated ADAMTS 13 variants. The type and position of amino acid variations were confirmed through nucleotide sequence analysis of the corresponding DNA.

[Table 1]

| Nucleotide sequences of primers for error-prone PCR | |
|---|---|
| **pPrimer** | **Sequence (5'→ 3')** |
| MDTCS-Forward | CACCGGTCTCNTAGAGCTGCTGGCGGCAT |
| MDTCS-Reverse | GGCTGGTCTCNCCCAAGCCTGCCGAGGCTTA |
| S-forward | CACCGGTCTCATAGAAGAGAATACGTGACCTTCCTGAC |
| S-reverse | GGCTGGTCTCACCCAGTATTCCTCGCCGTATCTTCTGTA |

*Transient expression of human ADAMTS13 variants*

For the expression of the proteins of ADAMTS 13 variants, Expi293F™ cells (Thermo Fisher, Cat. No. A14527) were prepared to a concentration of $3 \times 10^6$ cells/mL by diluting in the Expi293 Expression Medium. In the first tube, 2.5 μg of plasmid DNA was diluted in the opti-MEM I Reduced Serum Medium (Thermo Fisher, Cat. No. 31985-070) to a final volume of 152.5 μL, and in the other tube, 8 μL of ExpiFectamine 293 and 140 μL of opti-MEM I Reduced Serum Medium were mixed and allowed to stand for 5 minutes. 152.5 μL of the diluted plasmid DNA was added to the mixed solution in the second tube and allowed to stand for 20 minutes. 2.5 mL of the prepared cells was aliquoted into each well of the 24 deep well plates. The mixture of ExpiFectamine 293 and plasmid DNA prepared in advance was dispensed into the cells and cultured with shaking at 225 rpm in an incubator at 37°C and $CO_2$ conditions. After 24 hours, 15 μL of ExpiFectamine transfection enhancer 1 and 150 μL of enhancer 2 were added thereto and cultured for 5 days. On the 6th day, the culture was spun down at 300 rcf for 5 minutes to collect the supernatant and the resultant was used for analysis for selection of variants.

*Construction of human and mouse ADAMTS13 neutralizing antibodies*

In order to construct ADAMTS 13 neutralizing antibodies that bind to different domains, 16 kinds of Fabs having excellent binding ability to ADAMTS 13 were selected using a full-length recombinant human ADAMTS 13 (R&D Systems, Cat. No. 6156-AD) as an antigen and PLATINUM® phage library of Human Combinatorial Antibody Library (HuCAL, Bio-Rad), and among them, six kinds of antibodies (Ab60, Ab61, Ab64, Ab65, Ab66, and Ab67) that bind to each of the MDTCS part and the C-terminal part were prepared as full-length antibodies. Ab3-01, Ab 4-16, and Ab 4-20, which are known to bind specifically to the S domain in the plasma of an aTTP patient, were also constructed as full-length antibodies and used for selection analysis of variants.

*Measurement of expression concentration of human ADAMTS13 variant*

In order to confirm the expression level of the ADAMTS13 variant protein present in a culture medium obtained through transient expression, ELISA was performed using an anti-ADAMTS13 antibody (Ab 66, GC Green Cross antibody). 100 $\mu$L of the anti-ADAMTS13 antibody diluted to 2 $\mu$g/mL in PBS (Lonza, 17-516Q) was dispensed per well in a 96-well EIA/RIA high bind microplate (Corning, Cat. No. 3590) and allowed to react at room temperature for 2 hours. Then, the microplate was washed three times using wash buffer (0.1 v/v% tween 20 in PBS). Blocking buffer (1 w/v% bovine serum albumin in PBS) was dispensed at 300 $\mu$L per well and reacted at room temperature for 2 hours, followed by washing three times with wash buffer. Recombinant human ADAMTS13 (R&D Systems, 6156-AD) was used as a standard sample, and the culture to be measured was diluted 16-fold or 32-fold with blocking buffer, and dispensed at 100 $\mu$L per well and reacted by stirring at 500 rpm for one hour at room temperature. After washing three times, an anti-6X His tag antibody (Abcam, Cat. No. Ab1187) was diluted 1: 10,000 and dispensed at 100 $\mu$L per well, followed by reaction by stirring at 500 rpm at room temperature for 1 hour. After washing 6 times, TMB (3,3',5,5'-tetramethylbenzidine) was dispensed at 100 $\mu$L per well and allowed to react at room temperature for 10 minutes. Thereafter, stop buffer (H$_2$SO$_4$) was dispensed at 100 $\mu$L per well, and the absorbance was measured at 450 nm using a microplate reader. After substituting the measured absorbance into the standard sample response curve and calculating the concentration, the dilution factor was calibrated to determine the final concentration.

*Measurement of expression concentrations of Fc fusion MDTCS fragments and variants*

Goat anti-human IgG Fc (Abcam, Cat. No. A3803) diluted to 2 $\mu$g/mL using PBS (Lonza, Cat. No. 17-516Q) was dispensed at 100 $\mu$L per well into a 96 well EIA/RIA high bind microplate (Corning, Cat. No. 3590) and allowed to react at room temperature for two hours. Then, the microplate was washed three times using wash buffer (0.1 v/v% tween 20 in PBS). Blocking buffer (1 w/v% bovine serum albumin in PBS) was dispensed at 300 $\mu$L per well and allowed to react at room temperature for two hours, followed by washing three times with wash buffer. As a standard sample, the Fc fusion MDTCS fragment protein whose concentration was secured was used, and the sample to be measured was diluted with blocking buffer to be included in the standard range. Thereafter, the resultant was dispensed at 100 $\mu$L per well and reacted by stirring at 400 rpm at room temperature for one hour. After washing three times, an anti-human IgG (Fc specific) peroxidase antibody (Sigma-aldrich, Cat. No. A0170) was diluted 1:10,000 and dispensed at 100 $\mu$L per well and reacted by stirring at 400 rpm at room temperature for one hour. After washing six times, TMB was dispensed at 100 $\mu$L per well and allowed to react at room temperature for 30 minutes. Thereafter, stop buffer (0.5 M H$_2$SO$_4$) was dispensed at 100 $\mu$L per well, and the absorbance was measured at 450 nm with a microplate reader.

*Measurement of activity of human ADAMTS13 variants*

In order to measure the activity of ADAMTS 13 variants, an experiment was performed using the manual of the Technozyme ADAMTS 13 activity kit (Technoclone, Cat. No. 5450701). A GST-vWF73 substrate was dispensed at 100 $\mu$L per well, allowed to react at room temperature for one hour, and then washed three times with wash buffer. As a standard sample, the calibrator in the kit was used, and as the culture to be measured, a stock solution or a 3-fold dilution thereof was added at 100 $\mu$L per well using heat-inactivated plasma and allowed to react at room temperature for 30 minutes. After washing three times with wash buffer, the conjugate was added at 100 $\mu$L per well, allowed to react at room temperature for one hour, and washed three times with wash buffer again. TMB color reagent was added at 100 $\mu$L per well and allowed to react for 30 minutes, and then, 100 $\mu$L of stop buffer was added to stop the reaction, and the absorbance was measured at 450 nm with an ELISA reader. In order to calculate the specific titer (IU/mg), the expression result ($\mu$g/mL) was applied to the activity value (IU/mL), and the relative activity (%) was calculated by substituting into the following equation using the specific titers of wild-type (WT) ADAMTS 13 and variants:

$$\text{Relative activity } (\%) = \text{specific titer of variants/specific titer of wild-type ADAMTS13} \times 100$$

*Confirmation of escaping rate of human ADAMTS13 neutralizing antibody*

In order to confirm the ability of each ADAMTS 13 variant for escaping ADAMTS 13 neutralizing antibodies, ELISA was performed using eight kinds of anti-ADAMTS13 antibodies (Ab3-01, Ab 4-16, Ab4 -20, Ab60, Ab61, Ab64, Ab65, and Ab 67, GC Green Cross). An anti-ADAMTS13 antibody diluted to 1-2 $\mu$g/mL using PBS (Lonza, Cat. No. 17-516Q) was added at 100 $\mu$L per well in a 96-well EIA/RIA high bind microplate (Corning, Cat. No. 3590) and allowed to react at room temperature for two hours. Then, the microplate was washed three times with wash buffer (0.1% tween 20 in PBS). Blocking buffer (1% BSA in PBS) was added at 300 $\mu$L per well and allowed to react at room temperature for two hours, and then washed three times with wash buffer. As a standard sample, the recombinant human ADAMTS 13 (R&D systems, Cat. No. 6156-AD) was used, and the culture to be measured was diluted with blocking buffer to 25-50 ng/mL, and dispensed at 100 $\mu$L per well and reacted by shaking at 500 rpm at room temperature for one hour. After washing three times, an anti-6X His tag antibody (Abcam, Cat. No. Ab1187) was diluted 1:10,000 and dispensed at 100 $\mu$L per well, and reacted by shaking at 500 rpm at room temperature for one hour. After washing six times, TMB was dispensed at 100 $\mu$L per well and allowed to react at room temperature for 10-20 minutes. Then, stop buffer ($H_2SO_4$) was dispensed at 100 $\mu$L per well, and the absorbance was measured at 450 nm using a microplate reader. The neutralizing antibody escaping rate was calculated such that the relative binding level compared to the wild-type ADAMTS 13 value, and then, the relative escaping rate was substituted into the following equation using the same:

$$\text{Escaping rate (\%)} = (1- \text{ binding ability of variants/binding ability of wild-type ADAMTS13}) \times 100$$

*Construction of MDTCS and Fc (IgG1-YTE)-conjugated MDTCS variant and protein production*

An expression vector in which IgG1-YTE (hinge-Fc) was conjugated to MDTCS or an MDTCS variant fragment was constructed and gene synthesis (Thermo Fisher Scientific, GeneArt® Gene Synthesis) was performed as shown in Table 2 below. Expression vectors were constructed by inserting each of the synthetic genes in Table 2 into the pMSID2 vector (constructed by GC Green Cross). CHO DG44(S) cells were subcultured according to the expected amount using CDM4CHO (GE, Cat. No. SH30557.02) medium at $3 \times 10^5$ cells/mL. On the day of transfection, live $3 \times 10^7$ cells were collected, suspended in OptiPro™ SFM (Gibco, Cat. No. 12309019), diluted to a final volume of $1 \times 10^7$ cells/mL (3 mL) and added into a 125 mL shake flask, and cultured in the $CO_2$ shaking incubator (37°C, 5% $CO_2$) at 140 rpm. 30 $\mu$g of the expression vector was mixed to a total volume of 1.5 mL using OptiPro™ SFM (tube 1). 90 $\mu$L of the transporter 5™ PEI (Polyscience, Cat. No. 26008-5) and 1,410 $\mu$L of the OptiPro™ SFM were mixed (tube 2). After adding the previously prepared tube 2 into the tube 1 and gently mixing, the mixture was allowed to react at room temperature for 20 - 30 minutes. 3 mL of the DNA:PEI complex was added dropwise to 3 mL of previously prepared CHO DG44(S) cells. The resultant was cultured at 140 rpm in a $CO_2$ shaking incubator (37°C, 5% $CO_2$), and 4 hours thereafter, 24 mL of CDM4CHO medium was added to 6 mL of the CHO DG44(S)/DNA:PEI complex and cultured under the same conditions. After 48 hours of incubation, the cell number and cell viability were measured, and the resultant was added into a centrifuge tube to a volume of $15 \times 10^5$ cells, centrifuged at 1,200 rpm for 5 minutes, and the supernatant was removed, and suspended in 30 mL of CDM4CHO medium containing 20 nM MTX, and then inoculated into a shake flask and cultured. Subculture was performed every 3 to 4 days until the cell viability was recovered to 90% or more. The final overexpressing cell line was cultured on a large scale and used for protein purification. For MDTCS protein purification, the prepared culture was centrifuged to remove the cells and then filtered using a sterile filter made of cellulose acetate (Sartobran P, 5235307H7-OO, Sartorius) after centrifuging the prepared culture solution. The filtered culture was concentrated and then purified using anion exchange chromatography (Q Sepharose Fast Flow, GE), multi-mode chromatography (hydroxyapatite, Bio-Rad), and affinity chromatography (Blue Sepharose, GE), and the MDTCS variant IgG1-YTE-conjugated protein was filtered such that the culture of the overexpressing cell line was centrifuged to remove the cells and then purified using an antibacterial filter made of cellulose acetate (Sartobran P, 5235307H7-OO, Sartorius). Purification was performed using the filtered culture using affinity chromatography (Protein A, MabSelect SuRe, GE Healthcare) and size exclusion chromatography. It was confirmed that the purified protein had a purity of 95% or more.

[Table 2]

| Amino acid sequence of MDTCS-IgG1-YTE fusion | |
| --- | --- |
| Hinge | IgG 1 |
| Fc(CH2-CH3) | IgG4 (YTE) |
| Amino Acid Sequence | MHQRHPRARCPPLCVAGILACGFLLGCWGPSHFQQSCLQALEP QAVSSYLSPGAPLKGRPPSPGFQRQRQRQRRAAGGILHLELLV AVGPDVFQAHQEDTERYVLTNLNIGAELLRDPSLGAQFRVHL VKMVILTEPEGAPNITANLTSSLLSVCGWSQTINPEDDTDPGHA DLVLYITRFDLELPDGNRQVRGVTQLGGACSPTWSCLITEDTG FDLGVTIAHEIGHSFGLEHDGAPGSGCGPSGHVMASDGAAPRA GLAWSPCSRRQLLSLLSAGRARCVWDPPRPQPGSAGHPPDAQ PGLYYSANEQCRVAFGPKAVACTFAREHLDMCQALSCHTDPL DQSSCSRLLVPLLDGTECGVEKWCSKGRCRSLVELTPIAAVHG RWSSWGPRSPCSRSCGGGVVTRRRQCNNPRPAFGGRACVGAD LQAEMCNTQACEKTQLEFMSQQCARTDGQPLRSSPGGASFYH WGAAVPHSQGDALCRHMCRAIGESFIMKRGDSFLDGTRCMPS GPREDGTLSLCVSGSCRTFGCDGRMDSQQVWDRCQVCGGDN STCSPRKGSFTAGRAREYVTFLTVTPNLTSVYIANHRPLFTHLA VRIGGRYVVAGKMSISPNTTYPSLLEDGRVEYRVALTEDRLPR LEEIRIWGPLQEDADIQVYRRYGEEYGNLTRPDITFTYFQPKPR QA<u>EPKSCDKTHTCPPCP</u>*APEFLGGPSVFLFPPKPKDTLYITREPE VTCVVVDVSQEDPEVQFNWYVDGVEVHNAKTKPREEQFNSTYRV VSVLTVLHQDWLNGKEYKCKVSNKGLPSSIEKTISKAKGQPREPQV YTLPPSQEEMTKNQVSLTCLVKGFYPSDIAVEWESNGQPENNYKTT PPVLDSDGSFFLYSRLTVDKSRWQEGNVFSCSVMHEALHNHYTQK SLSLSLGK* |
| *Black: MDTCS/ Underline: Hinge/ Italic: Fc | |

*Confirmation of pharmacokinetics of MDTCS-hinge-Fc fusion*

In order to compare the duration in the blood and pharmacokinetic parameters of the MDTCS-IgG1-YTE (hinge-Fc) fusion, 44 or 52 C57BL/6 mice (4 mice/blood collection time) were used in each group. After injecting 160 IU/kg of MDTCS (control group) and 3 kinds of materials, in which IgG1-YTE is fused to MDTCS variants (test group), into the tail vein, the blood samples were collected at 0.083, 0.25, 0.5, 1, 2, 4, 8, 12, 24, 48, 72, 96, and 168 hours. 500 µL of blood was collected from the heart through a syringe containing 10% sodium tri-citrate (i.e., an anticoagulant), and centrifuged at 2,000 × g at 4°C for 20 minutes to separate the supernatant, and thereby the plasma was obtained. The activity of the MDTCS variant-hinge-Fc present in the plasma was measured according to the manual of Technozyme ADAMTS13 activity kit (Technoclone, Cat. No. 5450701). In addition, an activity assay was performed for the plasma obtained from the 44 mice to obtain an average value (0.0772 IU/mL) of the murine ADAMTS13 activity in the mouse plasma. By removing the corresponding value from the measured value of activity of the PK study sample, the murine ADAMTS 13 activity inherent in the mouse plasma could be excluded and only the result of activity of the administered material was obtained. The data was analyzed through non-compartmental analysis using a native pooled method and WINNONLIN. $t_{1/2}$ indicates the blood half-life of the drug, $AUC_{Inf}$ indicates the area under the activity graph from 0 to infinity time, and mean residence time (MRT) indicates the average residence time of the drug molecule in the body.

*Confirmation of ADAMTS13 binding site of neutralizing antibodies*

Nucleic acid sequences of six kinds of ADAMTS 13 in which full-length wild-type or C-terminal domains were sequentially deleted were cloned into pcDNA3.1 V5-His B vector (Invitrogen, Cat. No. V81020). The constructed DNA was immunoprecipitated by adding Protein A-Sepharose 4B using the culture obtained through the above-mentioned transient expression method, followed by Western blotting. Specifically, three kinds of 30 nM ADAMTS13 neutralizing antibodies (Ab4-16, Ab66, and Ab67) were added to normal control plasma (HemosIL, Cat. No. 00020003110). After adding 10 mL of plasma containing neutralizing antibodies and a culture containing 50 ng of each fragment protein of ADAMTS 13 together, 500 µL of binding buffer (50 mM Tris-HCl pH 8.0, 150 mM NaCl, 1% Triton X-100, and 1% BSA) was added thereto, and allowed to react at 4°C for 24 hours. After completion of the reaction, 20 µL of Protein A-Sepharose 4B resin (Thermo Fisher Scientific, Cat. No. 101041) was added thereto as a 50% slurry and allowed to react at 4°C for 4 hours. The resin was collected by centrifugation at 845 rcf for 1 minute, and then washed three times with wash buffer (50 mM Tris-HCl pH 8.0, 150 mM NaCl, 1% Triton X-100). The proteins bound to the resin was eluted through an SDS-sample buffer (Novex, Cat. No. NP0007) containing 10% β-mercaptoethanol and heated at 95°C for 5 minutes, followed by SDS-PAGE and anti-immunoblotting with the V5 antibody (Invitrogen, Cat. No. R960-25).

*PoC evaluation of variants in mouse model with aTTP mimic disease*

The mouse model with aTTP mimic disease was established by treating ADAMTS 13 KO mice with a human ADAMTS 13 neutralizing antibody. ADAMTS 13 KO mice were prepared by Macrogen by way of inducing large deletions and frameshift variations between exons 1 and 6 of the ADAMTS 13 gene using the CRISPR/Cas9 system. Nine kinds of ADAMTS13 neutralizing antibodies (Ab3-01, Ab4-16, Ab4-20, Ab60, Ab61, Ab64, Ab65, Ab66, and Ab67), which were mixed at an equal ratio, were administered intravenously to 12-25 week old ADAMTS 13 KO mice at a dose of 0.54 mg/kg. After 15 minutes, the control material (MDTCS-Fc) or five kinds of variants (1C03, 2B02, 5C09, 7A02, and DM2) were administered at a dose of 5,000 IU/kg or 7,000 IU/kg. The blood sample was collected 6 hours after the administration to measure ADAMTS 13 activity.

In order to confirm whether the recovery of ADAMTS 13 activity and hematological properties shown in aTTP model mice are improved by the administration of the DM2 variant, nine kinds of ADAMTS 13 neutralizing antibodies (Ab3-01, Ab4-16, Ab4-20, Ab60, Ab61, Ab64, Ab65, Ab66, and Ab67) which were mixed at an equal ratio, were administered intravenously to 12-25 week old ADAMTS 13 KO mice at a dose of 0.54 mg/kg. After 15 minutes, the control material (MDTCS-Fc) or variant DM2 was administered at a dose of 5,000 IU/kg, 7,000 IU/kg, or 14,000 IU/kg. After administration of 2,000 IU/kg of recombinant human vWF (VEYVONDI) 30 minutes after the administration of neutralizing antibodies, 700 µL of blood was collected 6 hours thereafter, and 300 µL of the blood was added into an EDTA tube (BD Medical, REF365974) and the other 300 µL was added into an SST tube (BD Medical, REF365967), placed at room temperature for 30 minutes, and then centrifuged at 4°C at 3,000 rpm for 15 minutes to separate the serum. The remaining blood was mixed with sodium citrate in a 9:1 ratio (blood: sodium citrate), and the plasma was separated and used to analyze the residual activity of human ADAMTS 13 in plasma. While the whole blood in the EDTA tube was subjected to general blood tests such as platelets (PLT) using a hemocytometer (ADVIA 2120i, Siemens), the serum separated in the SST tube was used to measure lactate dehydrogenase (LDH) using a blood biochemical analyzer (Hitachi, 7180). Statistical analysis used one-way ANOVA test with the Tukey test.

*PoC evaluation of variants in mouse model with cTTP disease*

The mouse model with cTTP disease was established using ADAMTS 13 KO mice. 12-25 week old ADAMTS 13 KO mice were administered intravenously with a vehicle or DM2 variant at a dose of 20 IU/kg, 60 IU/kg, 180 IU/kg, and 360 IU/kg, and 15 minutes thereafter, recombinant human vWF (VEYVONDI) was administered at a dose of 2,000 IU/kg, and the blood sample was collected 6 hours thereafter. While the whole blood was used for general blood tests including the measurement of the platelet level, the serum was used for the measurement of the LDH level, and the plasma was used for the analysis of human ADAMTS 13 activity.

**Experiment results**

*Construction of human* ADAMTS13 *variants using random mutagenesis*

In the case of ADAMTS 13 neutralizing antibodies possessed by aTTP patients, it has been reported that the proportion of binding to MDTCS among various domains of ADAMTS 13 is relatively very high being more than 97%, and that the S domain is the most prevalent binding site (Tersteeg et al, 2016; Klaus *et al.*, 2004; Leken *et al.*, 2005). Based on the above, each library was prepared by performing error-prone PCR so that variations could occur targeting the MDTCS part or the S domain (FIG. 1a). As a result of obtaining DNA for 384 ADAMTS 13 variants from each library and analyzing the nucleotide sequences, the total variation rate in the library of the MDTCS part was 72.9%, and specifically, silent mutation or non-mutation showed a rate of 23.7%, empty vectors or poor sequences 3.1%, nonsense mutation 8.9%, frameshift mutation 4.9%, and missense variation 59.1% (FIG. 1b). In the case of the S domain library, the overall mutation rate was 50.8%, and silent mutation or non-mutation accounted for 44.5%, empty vectors or poor sequences 4.7%, nonsense mutation 2.3%, frameshift mutation 3.1%, and missense mutation 45.3% (FIG. 1b). A selection experiment was performed using about 500 variants, excluding those variants which showed silent or non-mutation, empty vectors or poor sequences, and nonsense mutation among the above mutations.

*Preparation of ADAMTS13 neutralizing antibody and confirmation of binding site*

To confirm whether a variant having a mutation in the MDTCS region or the S domain can escape the neutralizing antibodies of ADAMTS 13, with regard to the antibodies that recognize different epitopes for human ADAMTS 13, Fab was constructed using the HuCAL system (Bio-Rad), and 16 kinds having excellent binding ability to human ADAMTS 13 were selected. Ab 66 and Ab 67, which were confirmed to specifically bind to different domains among the 16 kinds, were produced in the form of a full-length antibody. Since Ab 4-16 was known to specifically bind to the S domain in the plasma of the aTTP patient, it was produced in the form of a full-length antibody (Casina *et al.*, 2015), and the binding region of each antibody was, as shown in FIG. 2c, such that Ab 4-16 specifically binds to the S domain, Ab 67 to the MDTCS region, and Ab 66 to the C-terminal end (TSP-2 to TSP-8 domains).

*Setting selection criteria for variants*

The evaluation of the variants was conducted by analyzing the relative results with regard to the non-mutation or silent mutation variants (hereinafter, wild-type clones) having the same amino acid sequence as the wild-type ADAMTS 13, among the variants constructed through random mutagenesis. A total of 59 wild-type clones were analyzed for activity and binding affinities for Ab 4-16 and Ab 67, and the analysis results of each wild-type clone were indicated relative to the results of the wild-type ADAMTS 13 construct, and are shown in FIG. 3. As can be seen in FIG. 3, the escaping rates and relative activity with respect to wild-type ADAMTS 13 (59 kinds), mutated ADAMTS13 (304 kinds), and selected ADAMTS13 (26 kinds) containing a conservative amino acid substitution for Ab 4-16 and Ab 67 antibodies are shown. The wild-type clone showed a relative difference in result with the wild-type ADAMTS 13 construct despite no variation, and among the mutated ADAMTS13 (304 kinds), 26 kinds of ADAMTS 13 mutants having a specific value of escaping rate or a value greater than or equal to relative activity were selected as shown in Table 3 below.

Specifically, as for the mutated ADAMTS 13, the escaping rate for Ab 4-16 was in the range of -29.5% to 33.4%, the escaping rate for Ab 67 was in the range of -24.4% to 30.5%, and the relative activity was shown to be in the range of 62.7% to 128.9% (Table 3). As a result of applying the normal distribution using the three-sigma rule, the escaping rate for Ab 4-16 was -34.1% to 38.5%, the escaping rate for Ab 67 was -38.5% to 42.3%, and the relative activity was in the range of 47.0% to 145.2%, and thus it was predicted that almost all WT clones would fall within this distribution. Therefore, in the selection of variants, the selection criteria were applied as follows: the escaping rate of exceeding 38.5% for Ab 4-16, the escaping rate of exceeding 42.3% for Ab 67, or relative activity of 47.0% or more based on the maximum of three-sigma.

[Table 3]

| Range of escaping rate and relative activity for neutralizing antibody Ab4-16 or Ab67 | | | |
|---|---|---|---|
| Category | Escaping Rate for Ab 4-16 | Escaping Rate for Ab 67 | Relative Activity |
| Minimum | -29.5% | -24.4% | 62.7% |
| Maximum | 33.4% | 30.5% | 128.9% |
| Average | 2.2% | 1.9% | 96.1% |
| Standard Deviation | 12.1% | 13.5% | 16.4% |
| Mean - 3 Standard Deviation (Mean -3σ) | -34.1% | -38.5% | 47.0% |
| Mean + 3 Standard Deviation (Mean +3σ) | 38.5% | 42.3% | 145.2% |

*Selection of variants which evade ADAMTS13 neutralizing antibody and have activity higher than or equal to the level of wild-type ADAMTS13*

After transfection of WT clones and variants with amino acid variations into cells, the amount of proteins present in the culture was measured, and the binding affinity for neutralizing antibodies and activity assays were performed for 304 variants having an expression concentration of 50 ng/mL or more. The variants having amino acid variations were shown to have various distributions in binding affinity for neutralizing antibodies or relative activity compared to WT clones, and among them, 26 kinds of variants satisfying the selection criteria were finally selected (FIGS. 3a and 3b). Among the 26 variants, 18 kinds of variants were shown to have S domain variations, and in particular, 13 kinds of the variants having S domain variations (1C03, 1G07, 2B01, 2B02, 3B05, 3G04, 5C09, 5G08, 6B12, 7A02, 8C04, 8D01, and 8F01) were shown to have a high escaping rate for Ab 4-16, and 5 kinds of the variants (7E01, 7G08, 8C02, 8D01, and 8D05) were shown to have the characteristic of high relative activity (Table 4). Six kinds of variants were shown to have variations in the D domain, and five kinds among them (46, 3A06, 4C07, 4E11, and 4H07) showed the characteristic of a high escaping rate for Ab 67. In addition, 6 kinds of variants having a variation for the M domain, 2 kinds of variants having a variation for the C domain, and 2 kinds of variants having a variation for the T domain were identified. The repeated reproducibility of the results was confirmed through three repeated experiments for the 26 selected variants, and 12 kinds of variants that continuously maintained superiority compared to the WT clones even in repeated tests were selected as the subject for the *in vitro* potency test. Among the 12 kinds of variants, 1C03, 2B01, 2B02, 3B05, 5C09, 5G08, 7A02, and 8D01 were selected for their excellent Ab4-16 antibody escaping rate, and 4C07, 4E11 and 4H07 were selected for their excellent escaping rate for Ab67 antibody. Finally, 8D05 was selected for its excellent relative activity.

In order to confirm the ability to escape additional neutralizing antibodies in the 12 kinds of selected variants, the present inventors attempted to confirm whether it is also possible to escape even the neutralizing antibodies Ab4-20, Ab60, Ab61, Ab64, and Ab65 in addition to the Ab4-16 and Ab67 antibodies which were used for screening. Among the 12 kinds of variants, eight kinds of variants of the nine kinds of variants with amino acid variations in the S domain, except for 8D05, 1C03, 2B01, 2B02, 3B05, 5C09, 5G08, 7A02, and 8D01 showed an excellent escaping rate for Ab4-16 and Ab4-20, which were prepared based on the sequences of ADAMTS 13 autoantibodies possessed by aTTP patients, and these variants also showed excellent escaping ability for Ab60 and Ab61. In contrast, in the case of variants 4C07, 4E11, and 4H07 having amino acid variations in the D domain, the escaping rate for Ab67 was excellent (FIG. 4, Table 5).

[Table 4]

| Selection of 26 kinds of variants with excellent escaping ability or relative activity for Ab4-16 and Ab67 neutralizing antibodies | | | | | |
|---|---|---|---|---|---|
| Variant ID | Mutation Applied Domain | Variant Amino Acid Residues | Escaping Rate for Ab4-16 Antibody | Escaping Rate for Ab67 Antibody | Relative Activity |
| 46 | M,D | L85F P317H | 46.30% | 53.90% | 66.80% |
| 1C03 | S | S612Y | 48.00% | 10.40% | 104.60% |

(continued)

| Selection of 26 kinds of variants with excellent escaping ability or relative activity for Ab4-16 and Ab67 neutralizing antibodies | | | | | |
|---|---|---|---|---|---|
| Variant ID | Mutation Applied Domain | Variant Amino Acid Residues | Escaping Rate for Ab4-16 Antibody | Escaping Rate for Ab67 Antibody | Relative Activity |
| 1G07 | M,C,S | V282A<br>A465D<br>D672V | 41.40% | 57.90% | 63.80% |
| 2B01 | S | D635V | 87.40% | 5.40% | 62.20% |
| 2B02 | C,S | R452I<br>S612Y | 69.80% | 15.30% | 88.80% |
| 3A06 | M,D,T | R278I<br>A334T<br>D427N | 42.60% | 58.50% | 62.90% |
| 3B05 | S | P618S | 66.00% | 48.40% | 71.20% |
| 3E01 | M | T135I | 52.30% | 36.00% | 60.00% |
| 3G04 | M,S | V126M<br>A567S<br>E651D | 42.00% | 29.40% | 103.50% |
| 3H12 | T | N413D | -69.00% | -104.60% | 162.40% |
| 4C07 | D | A334V | -6.60% | 72.30% | 153.10% |
| 4E11 | D | A314T | 10.30% | 96.40% | 71.90% |
| 4H03 | M, D | F93V<br>K364R<br>E376D | 40.30% | 37.60% | 65.70% |
| 4H07 | D | N308K | 30.20% | 64.60% | 106.40% |
| 5C09 | S | S612F | 60.90% | -10.50% | 88.50% |
| 5G08 | S | Q656H | 41.40% | 26.00% | 91.90% |
| 6B12 | S | G607R | 43.90% | -101.60% | 93.80% |
| 7A02 | S | S612F<br>G624D | 57.00% | -80.50% | 128.50% |
| 7E01 | S | R589Q | -36.60% | -89.70% | 149.90% |
| 7G08 | S | Q650H<br>Q656R | -25.20% | -47.60% | 160.70% |
| 8C02 | S | I643F | -19.20% | -50.90% | 168.10% |
| 8C04 | S | I585N<br>Y658H | 51.30% | 34.80% | 69.00% |
| 8C12 | S | V630L<br>D654G<br>E664N | -12.00% | 73.20% | 48.10% |

(continued)

| Selection of 26 kinds of variants with excellent escaping ability or relative activity for Ab4-16 and Ab67 neutralizing antibodies | | | | | |
|---|---|---|---|---|---|
| Variant ID | Mutation Applied Domain | Variant Amino Acid Residues | Escaping Rate for Ab4-16 Antibody | Escaping Rate for Ab67 Antibody | Relative Activity |
| 8D01 | S | R589Q K608M M609L G624C I655V | 85.10% | -78.30% | 193.60% |
| 8D05 | S | P578L | -1.20% | -5.70% | 172.30% |
| 8F01 | S | I585M | 45.20% | 34.40% | 99.40% |

[Table 5]

| Confirmation of escaping ability and relative activity for single neutralizing antibodies in 12 kinds of selected variants | | | | | | | | | |
|---|---|---|---|---|---|---|---|---|---|
| Variant ID | Positionof Variation | Neutralizing Antibody Escaping Rate (%) | | | | | | | Relative Activity |
| | | Ab4-16 | Ab4-20 | Ab60 | Ab61 | Ab64 | Ab65 | Ab67 | |
| 1C03 | S612Y | 57.5% | 59.4% | 97.2% | 94.7% | 5.2% | -11.0% | 15.1% | 103.7% |
| 2B01 | D635V | 82.9% | 88.6% | 92.4% | 97.4% | 96.3% | 8.1% | 7.9% | 66.8% |
| 2B02 | R452I S612Y | 65.4% | 61.1% | 97.3% | 95.5% | 0.9% | -15.0% | 15.1% | 112.6% |
| 3B05 | P618S | 51.3% | -6.5% | 24.9% | 39.4% | 19.4% | -63.8% | 37.4% | 77.3% |
| 4C07 | A334V | 4.2% | -5.0% | -3.3% | -6.9% | -2.2% | -4.7% | 78.5% | 152.2% |
| 4E11 | A314T | 16.6% | -1.3% | 2.7% | 3.2% | 5.4% | -0.8% | 98.9% | 78.3% |
| 4H07 | N308K | 27.9% | 1.5% | 11.0% | 16.4% | 17.0% | 12.8% | 61.3% | 94.7% |
| 5C09 | S612F | 64.6% | 54.7% | 97.3% | 95.2% | 7.8% | -1.6% | 11.8% | 104.8% |
| 5G08 | Q656H | 47.0% | 7.2% | 32.1% | 46.2% | 27.4% | -51.4% | 30.3% | 95.9% |
| 7A02 | S612F G624D | 57.1% | 55.4% | 97.6% | 95.5% | 1.9% | -26.2% | -12.9% | 125.3% |
| 8D01 | R589Q K608M M609L G624C I655V | 90.1% | 98.5% | 95.2% | 94.1% | 20.0% | -95.9% | 7.3% | 127.0% |
| 8D05 | P578L | -9.3% | -43.1% | 3.2% | 15.9% | -0.9% | -116.4% | 11.5% | 154.7% |

Confirmation of neutralizing antibody escaping ability of Fc-attached MDTCS fragment variants

In a structure-function study, it was reported that among a total of 14 domains of ADAMTS 13, the MDTCS domain, in which the CUB2 domain was removed from the C-terminal TSP-2, was shown to have a metalloprotease function similar to ADAMTS 13 and is thus capable of cleaving VWF (Shelat *et al.*, 2005). This suggests that even only a MDTCS fragment, instead of a full-length ADAMTS 13, can function as a therapeutic agent for TTP disease, and that it can escape neutralizing antibodies present in the patient's plasma in a truncated form that bind to the C-terminal portion.
In addition, the present inventors attempted to further improve the structural stability of the MDTCS fragment and extend the half-life by attaching an Fc. The selected 12 kinds of variants were fragmented with MDTCS, and thereby Fc-attached variants were prepared, and DM1 and DM2 variants having two amino acid variations were additionally prepared by combining the selected variant amino acid residues. The final candidate materials were selected by measuring the escaping rate and relative activity of single or mixed neutralizing antibodies using the culture expressed in the cells by the above-mentioned method and a purification solution. As a result of measuring the escaping rates of binding to and relative activity for eight kinds of single neutralizing antibodies using the culture, 2B01, 3B05, 4H07, 5G08, 8D05, and DM1 were shown to escape all neutralizing antibodies at a similar level (FIG. 5, Table 6). 1C03, 2B02, 5C09, 7A02, 8D01, and DM2 showed excellent escaping rates of binding for 3-01 and Ab60. All of the six kinds of candidates were shown to have an amino acid variation in the S domain, and except for 8D01, have a variation in the 612th amino acid in common. In the cases of 4E11 and DM2, they were shown to have excellent escaping rates of binding to Ab67, and both were shown to have a variation in the 314th amino acid of the D domain. In the case of relative activity, DM1 showed the lowest of 57.9% and 1C03 showed the highest of 133.1%. All candidates except for the above two kinds showed relative activity of 78.2% to 125.1%, which was similar to that of the MDTCS-Fc control group (Table 6). In order to confirm the escaping ability of the candidate variants excluding 4C07 and 5C09, compared to the control group MDTCS-Fc, under the condition in which nine kinds of neutralizing antibodies (Ab3-01, Ab4-16, Ab4-20, Ab60, Ab61, Ab64, Ab65, Ab66, and Ab67) were mixed in the same ratio, 7.5 nM of the mixed neutralizing antibodies and 4 nM of each variant expressing culture were mixed, and allowed to react at room temperature for one hour to measure the residual activity. MDTCS-Fc was shown to maintain a residual activity of 3.5%, and 3B05, 4E11, 4H07, 5G08, and 8D05 were confirmed to have a residual activity of 1.24% to 2.42%, thus showing that the residual activity was lower than that of MDTCS-Fc (FIG. 6). In contrast, in the cases of 1C03, 2B01, 2B02, 7A02, 8D01, DM1, and DM2, the residual activity was maintained at 7.69% to 18.81%, thus confirming that the escaping ability by the mixed neutralizing antibodies was excellent compared to MDTCS-Fc (FIG. 6).

In order to confirm the neutralizing antibody escaping ability of the candidate material variants using the purifying solution, protein purification was performed in 12 kinds of cultures, excluding 4C07 and 5C09, using the Phytip system (protein A resin). The concentration of the purifying solution was confirmed through Fc ELISA, and it was confirmed that the target protein was eluted through silver staining. As a result of confirming the escaping rate of a single neutralizing antibody using the purifying solution, it was confirmed that most of the variants had similar tendencies to the results evaluated in the culture (FIG. 7). The 2B01 5G08, 8D05, and DM1 variants were shown to escape neutralizing antibodies for all eight kinds of neutralizing antibodies by 24.6% or more, and 1C03, 2B02, 7A02, 8D01, and DM2 showed excellent escaping rates of binding to 3-01 and Ab60 similar to the results of the culture. In the cases of 4E11 and DM2, it was confirmed that they had excellent escaping rates of binding to Ab67 to be the same as the results of the culture. The relative activity of each variant is shown in FIG. 7 and Table 7. As a result of measuring the residual activity under the condition of mixed neutralizing antibodies, it was confirmed that MDTCS-Fc maintained the residual activity of 3.76%, and it was confirmed that the 3B05, 4E11, 4H07, and 8D05 variants showed the residual activity of 2.05% to 3.50%, thus showing higher inhibition compared to MDTCS-Fc. In contrast, in the cases of 1C03, 2B01, 2B02, 5G08, 7A02, 8D01, DM1, and DM2, it was confirmed that the residual activity of 6.34% to 12.8% was maintained, thus confirming that the mixed neutralizing antibodies have excellent ability of escaping neutralizing antibodies compared to MDTCS-Fc (FIG. 8).

Based on the above results, comprehensively considering the relative activity or escaping rates for mixed neutralizing antibodies, 1C03, 2B02, 7A02, DM2, and 5C09, which is predicted to have excellent ability of escaping mixed neutralizing antibodies due to the variation on the 612th amino acid, were selected as five kinds of candidates for further study.

[Table 6]

| Confirmation of single neutralizing antibody escaping ability and relative activity of MDTCS fragments including 14 variants (culture medium conditions) | | | | | | | | | | |
|---|---|---|---|---|---|---|---|---|---|---|
| Varian t ID | Positio n of Variati on | Neutralizing Antibody Escaping Rate (%) | | | | | | | | Relativ e Activity |
| | | 3-01 | 4-16 | 4-20 | Ab60 | Ab61 | Ab64 | Ab65 | Ab67 | |
| 1C03 | S612Y | 100.0% | 2.0% | -11.0% | 91.3% | 3.9% | -5.9% | -16.9% | 1.1% | 133.1% |
| 2B01 | D635 V | 25.1% | 28.2% | 38.1% | 38.4% | 36.2% | 26.8% | 11.0% | 24.9% | 78.2% |
| 2B02 | R452I S612Y | 99.0% | 1.2% | 4.2% | 93.5% | 2.9% | -2.1% | -12.9% | 3.1% | 116.5% |
| 3B05 | P618S | 35.3% | 18.9% | 25.5% | 23.6% | 19.1% | 21.3% | 17.9% | 17.8% | 92.7% |

(continued)

| Confirmation of single neutralizing antibody escaping ability and relative activity of MDTCS fragments including 14 variants (culture medium conditions) | | | | | | | | | | |
|---|---|---|---|---|---|---|---|---|---|---|
| Variant ID | Position of Variation | Neutralizing Antibody Escaping Rate (%) | | | | | | | | Relative Activity |
| | | 3-01 | 4-16 | 4-20 | Ab60 | Ab61 | Ab64 | Ab65 | Ab67 | |
| 4C07 | A334 V | -5.9% | -11.2% | -9.6% | -11.3% | -3.3% | -12.4% | -14.6% | 14.5% | 118.2% |
| 4E11 | A314T | 15.3% | 7.3% | 11.9% | 8.1% | 9.0% | 9.7% | 8.8% | 83.5% | 89.7% |
| 4H07 | N308 K | 36.1% | 20.4% | 25.8% | 19.5% | 22.5% | 25.1% | 21.0% | 53.8% | 93.6% |
| 5C09 | S612F | 100.0% | 15.7% | 18.7% | 100.0% | 12.4% | 13.0% | 0.5% | 8.5% | 108.2% |
| 5G08 | Q656 H | 48.7% | 38.3% | 41.2% | 44.2% | 41.9% | 37.2% | 27.8% | 47.6% | 85.4% |
| 7A02 | S612F G624 D | 100.0% | 7.5% | 17.4% | 97.5% | 11.2% | 7.2% | -7.2% | 12.3% | 125.1% |
| 8D01 | R589Q K608 M M609 L G64C I655V | 100.0% | 38.9% | 49.1% | 71.2% | 31.6% | 36.3% | 18.1% | 41.8% | 78.4% |
| 8D05 | P578L | 39.6% | 29.9% | 38.3% | 32.6% | 32.4% | 32.5% | 21.5% | 43.7% | 82.1% |
| DM1 | A314T D635 V | 52.3% | 48.2% | 56.9% | 60.6% | 54.7% | 51.6% | 38.1% | 86.8% | 57.9% |
| DM2 | A314T S612F | 100.0% | 12.0% | 22.0% | 95.1% | 21.6% | 13.0% | 7.3% | 84.8% | 97.7% |

[Table 7]

| Confirmation of single neutralizing antibody escaping ability and relative activity of MDTCS fragments including 14 kinds of variants (purifying solution condition) | | | | | | | | | | |
|---|---|---|---|---|---|---|---|---|---|---|
| Variant ID | Position of Variation | Neutralizing Antibody Escaping Rate (%) | | | | | | | | Relative Activity |
| | | 3-01 | 4-16 | 4-20 | Ab60 | Ab61 | Ab64 | Ab65 | Ab67 | |
| 1C03 | S612Y | 100.0% | 15.6% | 21.1% | 100.0% | 16.3% | 7.3% | -7.1% | 10.9% | 81.4% |
| 2B01 | D635V | 46.0% | 37.8% | 49.6% | 65.5% | 43.7% | 49.7% | 28.5% | 28.2% | 56.3% |
| 2B02 | R452I | 100.0% | 8.4% | 15.0% | 100.0% | 10.3% | 4.4% | -9.0% | 0.2% | 91.1% |
| 2B02 | S612Y | 100.0% | 8.4% | 15.0% | 100.0% | 10.3% | 4.4% | -9.0% | 0.2% | 91.1% |
| 3B05 | P618S | 25.5% | 11.9% | 13.3% | 14.9% | 10.5% | 13.8% | 7.9% | 10.2% | 83.7% |
| 4E11 | A314T | 19.6% | 6.2% | 11.5% | 15.7% | 1.8% | 8.8% | 12.6% | 84.4% | 77.5% |
| 4H07 | N308K | 32.6% | 12.80% | 16.8% | 29.0% | 9.4% | 16.9% | 17.0% | 47.1% | 77.5% |
| 5G08 | Q656H | 89.6% | 64.5% | 70.2% | 81.9% | 57.90% | 68.9% | 59.0% | 37.7% | 18.3% |
| 7A02 | S612F G624D | 100.0% | 10.4% | 12.8% | 100.0% | 8.5% | 3.4% | -7.3% | 14.4% | 97.8% |
| 8D01 | R589Q K608M M609L G64C I655V | 100.0% | 29.5% | 48.2% | 91.7% | 20.5% | 17.1% | 14.2% | 31.5% | 65.1% |
| 8D05 | P578L | 51.0% | 27.4% | 35.1% | 49.5% | 27.2% | 33.8% | 29.9% | 46.0% | 56.3% |
| DM1 | A314T D635V | 50.2% | 34.0% | 54.0% | 66.50% | 45.3% | 52.9% | 24.6% | 85.1% | 50.3% |
| DM2 | A314T S612F | 100.0% | 16.2% | 24.2% | 100.0% | 24.0% | 11.5% | 11.0% | 86.5% | 73.1% |

EP 4 249 594 A1

Ability of escaping binding and relative activity of eight kinds of single neutralizing antibodies were measured in the culture expressed in cells by constructing DNA in which IgG1-YTE was conjugated to the finally selected five kinds of MDTCS variant fragments. As a result, 1C03, 2B02, 5C09, and 7A02 showed excellent escaping rates of binding to Ab3-01 and Ab60, and in the case of DM2, it was confirmed that the escaping rates of binding to Ab3-01, Ab60, and Ab67 were excellent (FIG. 10). The relative activity was 98.6-127.7% being similar to that of MDTCS-IgG1-YTE at (FIG. 10). In order to confirm the escaping ability of five kinds of variant materials compared to the control group MDTCS-IgG1-YTE under the condition where nine kinds of neutralizing antibodies were mixed in the same ratio, the mixed neutralizing antibodies (nine kinds) and each variant expressing culture were mixed, allowed to react at room temperature for one hour, and the residual activity was measured. In the case of MDTCS- IgG 1- YTE, the residual activity was maintained at 5.5%, and in the case of the five kinds of materials, the residual activity was maintained at 8.18% to 13.42%, thus confirming that the escaping ability by the neutralizing antibodies was excellent compared to the control group (FIG. 11).

The protein was purified using the Phytip system (protein A resin) from the expression culture in which IgG1-YTE was conjugated to the five kinds of MDTCS variant fragments above, and the residual activity and specific titer of the mixed neutralizing antibodies were measured in the purifying solution. The concentration of the corresponding purifying solution was confirmed through Fc ELISA, and the elution of the target protein was confirmed through silver staining. As a result of measuring the residual activity under the condition of mixed neutralizing antibodies, it was confirmed that MDTCS-IgG1-YTE was maintained at 0.4%, and the residual activity of 1C03, 2B02, 5C09, 7A02, and DM2 were each maintained at 5.7%, 1.7%, 8.8%, 2.6%, and 7.9%, thus confirming that the mixed antibody escaping ability was superior to that of MDTCS- IgG 1- YTE. As a result of measuring specific titers, it was confirmed that the four kinds of variants except for 7A02 (10,288 IU/mg) showed specific titers of 19,091 IU/mg to 22,379 IU/mg similar to the control group (18,030 IU/mg) (FIG. 12). As a result, it was confirmed that the IgG1-YTE-conjugated materials of the five variant fragments had excellent escaping ability in the mixed neutralizing antibodies compared to the control group, and that the four kinds of variants except for 7A02 had specific titers similar to the control group.

*Evaluation of whether half-life of MDTCS fragment variants has increased by Fc attachment*

In order to determine whether Fc attached to MDTCS (IgG1-YTE) will actually increase the half-life, a pharmacokinetic analysis was performed in mice. For this purpose, MDTCS or MDTCS, to which IgG1-YTE is attached, and four final kinds of candidates (1C03, 5C09, 7A02, and DM2) variant fragment proteins were administered to the tail vein of the mice and then plasma was obtained for each set time. These materials were administered to a concentration of 160 IU/kg based on the specific titer of each material and the activity of the materials remaining in the plasma obtained for each set time was measured through an activity assay. The obtained results were summarized by the naive pooled method, and a pharmacokinetic analysis was performed using the noncompartmental analysis method. The half-life of MDTCS was measured to be 2.898 hours, MDTCS-IgG1-YTE was 11.51 hours, and each variant showed a half-life of 5.184 to 9.902 hours. Compared to the control MDTCS, the half-life was extended by 1.79 to 3.97 times by IgG1-YTE conjugation. In addition, the mean residence time (MRT) value indicating the average residence time of the material by IgG1-YTE conjugation was 7.189 to 11.67 hours, thus indicating an increased residence time compared to 3.743 hours (Table 8, FIG. 9). As a result, it was confirmed that the IgG1-YTE fusion was effective in maintaining activity through blood half-life and average duration *in vivo*.

[Table 8]

| Pharmacokinetic Results | | |
|---|---|---|
| Candidate Material | Parameters of PK Analysis | | |
| | $t_{1/2}$, Terminal (hr) | $AUC_{Inf}$ (IU*hr/mL) | MRT (hr) |
| MDTCS | 2.898 | 5.467 | 3.743 |
| MDTCS-IgG1-YTE | 11.51 | 16.89 | 11.67 |
| 1C03-IgG1-YTE | 6.087 | 12.33 | 8.695 |
| 5C09-IgG 1- YTE | 5.184 | 12.06 | 7.982 |
| 7A02-IgG1-YTE | 9.902 | 14.49 | 10.35 |
| DM2-IgG1-YTE | 5.986 | 11.39 | 7.189 |

As described above, the present inventors have discovered 26 kinds of novel variants that either escape ADAMTS 13 neutralizing antibodies binding to the MDTCS region or the S domain or exhibit activity greater than or equal to wild-type ADAMTS 13. Among these, 12 kinds having most excellent escaping ability or remarkably excellent comparative activity for the nine kinds of neutralizing antibodies were selected, and then constructed MDTCS fragments essential for vWF cleavage while capable of efficiently escaping the neutralizing antibodies binding to the C-terminus, thereby identifying variants with a significantly improved rate of escaping autoantibodies binding to D, C or S domains. The variants of the present invention can be usefully used as an effective pharmacological ingredient with improved stability and durability of physiological activity by increasing the blood half-life through the IgG1-YTE conjugation.

*Confirmation result of variant PoC in aTTP mimic disease mouse model*

In order to select the final candidate materials from the established aTTP-mimic mouse model, the control material (MDTCS- IgG 1- YTE) or selected five variant candidate materials (1C03-IgG1-YTE, 2B02-IgG1-YTE, 5C09-IgG1-YTE, 7A02-IgG1-YTE, and DM2-IgG1-YTE) were administered, and the rate of escaping a neutralizing antibody was evaluated (FIG. 13a). Among the five kinds of variant candidate materials, DM2-IgG1-YTE showed the highest human ADAMTS 13 residual activity at both doses of 5,000 IU/kg and 7,000 IU/kg (FIG. 13b). The DM2-IgG1-YTE material, which showed the best neutralizing antibody escaping rate, was finally selected and administered at various concentrations to confirm the ability to maintain residual activity of human ADAMTS 13 and the degree of alleviation of clinical symptoms (FIG. 14a); as a result, it was confirmed that the degree of alleviation increased as the DM2 dose increased, and it was observed that the improvement of platelet and LDH levels was relatively high by DM2-IgG1-YTE administration compared to MDTCS-IgG1-YTE administration in the 7,000 IU/kg administration group (FIG. 14b). In consistent with the improvement of platelet and LDH levels, as a result of the human ADAMTS 13 activity test, it was possible to observe an increase in the residual activity proportional to the administered dose of the control material or candidate material (FIG. 14b). As a result of observation of clinical symptoms, it was confirmed that mortality or hematuria shown in the aTTP-mimic mouse model was reduced by the administration of the control material or candidate material. In particular, in the case of the DM2-IgG1-YTE treatment group, there were no deaths at any dose, and no subjects with hematuria symptoms were observed when administered at a dose of 7,000 IU/kg or more (FIG. 14c). When administered at a dose of 7,000 IU/kg, the average residual activity was 0.32 IU/mL of DM2-IgG1-YTE, which was 9.6 times higher than that of 0.03 IU/mL of MDTCS- IgG 1- YTE, and it was determined that the differences in observation results of clinical symptoms was due to such difference in residual activity. As a result of a general blood test, clinical chemical test, and observation of clinical symptoms, it was confirmed the administrations of MDTCS-IgG1-YTE and DM2-IgG1-YTE tended to improve the clinical symptoms of aTTP in the aTTP-mimic mouse model, and through this, it was possible to confirm the *in vivo* PoC. In particular, it was confirmed that when administered at a dose of 7,000 IU/kg, the DM2-IgG1-YTE administration showed an excellent improvement effect compared to the MDTCS-IgG1-YTE administration.

*Result of confirmation of variant PoC in cTTP disease mouse model*

The presence of an improvement in hematological and clinical symptoms appearing in TTP disease and the degree of recovery of human ADAMTS 13 activity in the cTTP mouse model, was confirmed by using DM2-IgG1-YTE, which showed the most excellent effect among the five kinds of variant candidates in the aTTP minic mouse model (FIG. 15a). It could be confirmed that the improvement of platelet and LDH levels occurred in a concentration-dependent manner according to an increase of the dose of DM2-IgG1-YTE, and a significant increase in platelets was observed in the group administered with DM2-IgG1-YTE 180 at 360 IU/kg compared to the control group, and in particular, in the case of the group administered at 360 IU/kg, a recovery comparable to the normal level was shown (FIG. 15b). In the case of LDH level, it was confirmed that LDH was recovered to a level similar to that of the control group in the group administered at 180 IU/kg (FIG. 15b). In the case of ADAMTS 13 activity, starting from the group administered with DM2-IgG1-YTE at a dose of 60 IU/kg group, an average activity of 0.1 IU/mL or more was shown, and in the group administered at a dose of 360 IU/kg, an average activity of 1.08 IU/mL was measured (FIG. 15b). Therefore, it was found that the DM2-IgG1-YTE variant effectively could improve clinical symptoms and restore ADAMTS 13 activity in the cTTP disease mice.

As the specific parts of the present invention have been described in detail above, these specific descriptions are only preferred embodiments to those of ordinary skill in the art, and it is clear that the scope of the present invention is not limited thereto. Accordingly, the substantial scope of the present invention will be defined by the appended claims and equivalents thereof.

**References**

1. Miesbach W, Menne J, Bommer M, Schonermarck U, Feldkamp T, Nitschke M, Westhoff TH, Seibert FS, Woitas R, Sousa R, Wolf M, Walzer S, Schwander B. Orphanet Journal of Rare Diseases (2019) 14:260. doi: 10.1186/s13023-019-1240-0.

2. Ercig B, Wichapong K, Reutelingsperger CPM, Vanhoorelbeke K, Voorberg J, Nicolaes GAF. Thromb Haemost. 2018;118(1):28-41. doi:10.1160/TH17-06-0404

3. Johanna A Kremer Hoving, Paul Coppo, Bernhard Lammle, Joel L Moake, Toshiyuki Miyata, Karen Vanhoorelbeke. Nat Rev Dis Primers. 2017 Apr 6; 3:17020. doi: 10.1038/nrdp.2017.20.

4. Genetic and Rare Diseases Information Center (GARD)-an NCATS Program. 10 October 2018

5. Tersteeg C, Verhenne S, Roose E, et al. Expert Rev Hematol. 2016; 9(2): 209-221. doi:10.1586/17474086.2016.1122515

6. Klaus C, Plaimauer B, Studt JD, et al. Blood. 2004; 103(12): 4514-4519. doi: 10.1182/blood-2003-12-4165

7. Luken BM, Turenhout EA, Hulstein JJ, Van Mourik JA, Fijnheer R, Voorberg J. Thromb Haemost. 2005; 93(2):267-274. doi:10.1160/TH04-05-0301

8. Veronica C. Casina, Wenbing Hu, Jian-Hua Mao, Rui-Nan Lu, Hayley A. Hanby, Brandy Pickens, Zhong-Yuan Kan, Woon K. Lim, Leland Mayne, Eric M. Ostertag, Stephen Kacir, Don L. Siegel, S. Walter Englander, and X. Long Zheng. Proc Natl Acad Sci USA. 2015 Aug 4; 112(31): 9620-5. doi: 10.1073/pnas.1512561112.

9. Suresh G Shelat, Jihui Ai, and X. Long Zheng. Semin Thromb Hemost. 2005 Dec; 31(6):659-72. doi: 10.1055/s-2005-925472.

**Claims**

1. An ADAMTS 13 (a disintegrin and metalloproteinase with a thrombospondin type 1 motif, member 13) variant protein comprising substitutions of one or more amino acid residues selected from the group consisting of the 85th, 93rd, 126th, 135th, 278th, 282nd, 308th, 314th, 317th, 334th, 364th, 376th, 413rd, 427th, 452nd, 465th, 567th, 578th, 585th, 589th, 607th, 608th, 609th, 612th, 618th, 624th, 630th, 635th, 643rd, 650th, 651st, 654th, 655th, 656th, 658th, 664th, and 672nd residues of SEQ ID NO: 1, or a functional fragment thereof.

2. The ADAMTS 13 variant protein or a functional fragment thereof of claim 1, wherein the variant protein of ADAMTS 13 is selected from the group consisting of each of the variant proteins comprising substitutions of the amino acid residues at the following positions:

    - the 85th and 317th residues; the 612th residue; two or more among the 282nd, 465th, and 672nd residues; the 635th residue; the 452nd and 612th residues; two or more among the 278th, 334th, and 427th residues; the 618th residue; the 135th residue; two or more among the 126th, 567th, and 651st residues; the 413th residue; the 334th residue; the 314th residue; two or more among the 93rd, 364th, and 376th residues; the 308th residue; the 656th residue; the 607th residue; the 612 and 624th residues; the 589th residue; the 650th and 656th residues; the 643rd residue; the 585th and 658th residues; two or more among the 630th, 654th, and 664th residues; four or more among the 589th, 608th, 609th, 624th, and 655th residues; the 578th residue; the 585th residue; the 314th and 635th residues; and the 314th and 612th residues.

3. The ADAMTS 13 variant protein or a functional fragment thereof of claim 1, wherein the 85th residue is substituted with Phe, the 93rd residue with Val, the 126th residue with Met, the 135th residue with Ile, the 278th residue with Ile, the 282nd residue with Ala, the 308th residue with Lys, the 314th residue with Thr, the 317th residue with His, the 334th residue with Thr or Val, the 364th residue with Arg, the 376th residue with Asp, the 413th residue with Asp, the 427th residue with Asn, the 452nd residue with Ile, the 465th residue with Asp, the 567th residue with Ser, the 578th residue with Leu, the 585th residue with Asn or Met, the 589th residue with Gln, the 607th residue with Arg, the 608th residue with Met, the 609th residue with Leu, the 612th residue with Phe or Tyr, the 618th residue with Ser, the 624th residue with Asp or Cys, the 630th residue with Leu, the 635th residue with Val, the 643rd residue with Phe, the 650th residue with His, the 651st residue with Asp, the 654th residue with Gly, the 655th residue with Val, the 656th residue with Arg or His, the 658th residue with His, the 664th residue with Asn, and the 672nd residue with Val, respectively.

4. A fusion protein comprising:

    (a) the variant protein of ADAMTS13 or a functional fragment thereof of any one of claims 1 to 3; and
    (b) an Fc region of the IgG4 immunoglobulin conjugated to (a) above.

5. The fusion protein of claim 4, wherein the Fc region comprises substitutions of one or more amino acid residues selected from the group consisting of the 22nd, 24th, and 26th residues of SEQ ID NO: 2.

6. The fusion protein of claim 5, wherein the 22nd residue is substituted with Tyr, the 24th residue with Thr, and the 26th residue with Glu, respectively.

7. The fusion protein of claim 5, further comprising a hinge region of an IgG1 immunoglobulin between said (a) and (b).

8. A nucleotide encoding the ADAMTS 13 variant protein or a functional fragment thereof according to any one of claims 1 to 3; or the fusion protein according to any one of claims 4 to 7.

9. A composition for preventing or treating thrombotic disease comprising the ADAMTS 13 variant protein or a functional fragment thereof according to any one of claims 1 to 3; or the fusion protein according to any one of claims 4 to 7; or the nucleotide of claim 8 as an active ingredient.

10. The composition of claim 9, wherein the thrombotic disease is thrombotic microangiopathy (TMA).

11. The composition of claim 10, wherein the thrombotic microangiopathy (TMA) is selected from the group consisting of thrombocytopenic purpura (TTP), hemolytic uremic syndrome (HUS), HELLP (Hemolysis, Elevated Liver enzymes, and Low Platelet count) syndrome, preeclampsia, and sickle cell disease.

12. A method for screening ADAMTS13 variants with an improved escaping rate for autoantibodies, comprising the following steps of:

   (a) preparing an ADAMTS13 variant, in which one or more amino acid residues selected from the group consisting of the 85th, 93rd, 126th, 135th, 278th, 282nd, 308th, 314th, 317th, 334th, 364th, 376th, 413rd, 427th, 452nd, 465th, 567th, 578th, 585th, 589th, 607th, 608th, 609th, 612th, 618th, 624th, 630th, 635th, 643rd, 650th, 651st, 654th, 655th, 656th, 658th, 664th, and 672nd residues of SEQ ID NO: 1 are substituted or deleted; and
   (b) contacting an autoantibody for ADAMTS13 with the ADAMTS13 variant prepared in step (a) above;
   wherein when the autoantibody binds to the ADAMTS 13 variant with an affinity lower than that for wild-type ADAMTS 13, the ADAMTS 13 variant is determined as an ADAMTS 13 variant with an improved escaping rate for autoantibodies.

13. The method of claim 12, wherein step (a) above is performed by substituting on or more amino acid residues selected from the group consisting of the 85th, 93rd, 126th, 135th, 278th, 282nd, 308th, 314th, 317th, 334th, 376th, 413rd, 427th, 465th, 567th, 578th, 585th, 607th, 609th, 612th, 624th, 630th, 643rd, 650th, 654th, 655th, 656th, 658th, and 672nd residues of SEQ ID NO: 1.

[FIG. 1a]

[FIG. 1b]

[FIG. 2a]

[FIG. 2b]

[FIG. 2c]

[FIG. 3a]

EP 4 249 594 A1

Ab 4-16

Relative activity (%)

O Wild-type ADAMTS13 (n=59)
● Mutated ADAMTS13 (n=304)
● Selected ADAMTS13 (n=26)

[FIG. 3b]

[FIG. 4a]

[FIG. 4b]

[FIG. 5a]

[FIG. 5b]

[FIG. 6]

[FIG. 7a]

[FIG. 7b]

[FIG. 8]

[FIG. 9]

[FIG. 10a]

[FIG. 10b]

[FIG. 11]

**NAb mixture treated condition**

[FIG. 12]

[FIG. 13a]

**9 kinds of
neutralizing antibodies**

0.54 mg/kg

**MDTCS-IgG1-YTE / 5 kinds of variants**

5,000 IU/kg
7,000 IU/kg

0            15 min            6 h

**whole blood
from heart
(n=6)**

[FIG. 13b]

[FIG. 14a]

**MDTCS-IgG1-YTE/**
**DM2-IgG1-YTE**

5,000 IU/kg

**9 kinds of**
**neutralizing antibodies**　　7,000 IU/kg　　**rh-vWF**

0.54 mg/kg　　14,000 IU/kg　　2,000 IU/kg

0　　15 min　　30 min　　6 h

whole blood
from heart
(n=6)

[FIG. 14b]

[FIG. 14c]

**Death**

**Hematuria**

WT + Vehicle
KO + Vehicle
KO + MDTCS-IgG1-YTE 5,000 IU/kg
KO + MDTCS-IgG1-YTE 7,000 IU/kg
KO + MDTCS-IgG1-YTE 14,000 IU/kg
KO + DM2-IgG1-YTE 5,000 IU/kg
KO + DM2-IgG1-YTE 7,000 IU/kg
KO + DM2-IgG1-YTE 14,000 IU/kg

[FIG. 15a]

**DM2-IgG1-YTE**
20~360 IU/kg

**rh-vWF**
2,000 IU/kg

0                    15 min                    6 h

**whole blood
from heart**
(n=6)

[FIG. 15b]

## INTERNATIONAL SEARCH REPORT

| International application No. |
| --- |
| **PCT/KR2021/014991** |

| A. | CLASSIFICATION OF SUBJECT MATTER |
| --- | --- |

**C12N 9/64**(2006.01)i; **C12N 15/85**(2006.01)i; **A61K 38/48**(2006.01)i; **A61P 7/02**(2006.01)i; **C07K 16/40**(2006.01)i; **G01N 33/573**(2006.01)i; **G01N 33/68**(2006.01)i

According to International Patent Classification (IPC) or to both national classification and IPC

| B. | FIELDS SEARCHED |
| --- | --- |

Minimum documentation searched (classification system followed by classification symbols)

C12N 9/64(2006.01); A61K 38/17(2006.01); A61K 38/48(2006.01); A61K 39/395(2006.01); A61K 9/00(2006.01); C07K 16/18(2006.01); C07K 16/46(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: ADAMTS13(a disintegrin and metalloproteinase with a thrombospondin type 1 motif, member 13), 치환(substitution), 변이(mutation), IgG4 면역글로불린(IgG4 immunoglobulin), Fc 영역(Fc region), 융합 단백질(fusion protein), 혈전성 혈소판 감소성 자반증(thrombocytopenic purpura, TTP), 자가항체(autoantibody)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | KR 10-2016-0016756 A (BAXALTA INCORPORATED et al.) 15 February 2016 (2016-02-15)<br>See paragraphs [0051]-[0087]. | 1,2,4 |
| Y | | 5-7 |
| A | | 3,12,13 |
| Y | KR 10-1941514 B1 (TEVA PHARMACEUTICALS AUSTRALIA PTY LTD.) 23 January 2019 (2019-01-23)<br>See paragraphs [0143] and [0362]; and figure 8. | 5-7 |
| A | US 2015-0044171 A1 (THE CHILDREN`S HOSPITAL OF PHILADELPHIA) 12 February 2015 (2015-02-12)<br>See abstract; and claims 1-17. | 1-7,12,13 |

| ☑ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **07 February 2022** | **07 February 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2021/014991** |

**C.    DOCUMENTS CONSIDERED TO BE RELEVANT**

| Category* | Citation of document, with indication, where appropriate, of the relevant passages | Relevant to claim No. |
| --- | --- | --- |
| A | JIAN, Cui et al. Gain-of-function ADAMTS13 variants that are resistant to autoantibodies against ADAMTS13 in patients with acquired thrombotic thrombovytopenic purpura. Blood. 30 January 2012 (online publication date), vol. 119, no. 16, pp. 3836-3843.<br>    See pages 3836-3842. | 1-7,12,13 |
| A | US 2019-0161746 A1 (BAXALTA INCORPORATED et al.) 30 May 2019 (2019-05-30)<br>    See abstract; and claims 8-26. | 1-7,12,13 |
| A | WO 2017-055908 A1 (NORIS, Marina et al.) 06 April 2017 (2017-04-06)<br>    See abstract; and claims 1-24. | 1-7,12,13 |

Form PCT/ISA/210 (second sheet) (July 2019)

**INTERNATIONAL SEARCH REPORT**

| International application No. |
|---|
| **PCT/KR2021/014991** |

| Box No. I       Nucleotide and/or amino acid sequence(s) (Continuation of item 1.c of the first sheet) |
|---|

1. With regard to any nucleotide and/or amino acid sequence disclosed in the international application, the international search was carried out on the basis of a sequence listing:

    a.    ☑   forming part of the international application as filed:

          ☑    in the form of an Annex C/ST.25 text file.

          ☐    on paper or in the form of an image file.

    b.    ☐   furnished together with the international application under PCT Rule 13*ter*.1(a) for the purposes of international search only in the form of an Annex C/ST.25 text file.

    c.    ☐   furnished subsequent to the international filing date for the purposes of international search only:

          ☐    in the form of an Annex C/ST.25 text file (Rule 13*ter*.1(a)).

          ☐    on paper or in the form of an image file (Rule 13*ter*.1(b) and Administrative Instructions, Section 713).

2.   ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

## INTERNATIONAL SEARCH REPORT

| International application No. |
| --- |
| **PCT/KR2021/014991** |

**Box No. II       Observations where certain claims were found unsearchable (Continuation of item 2 of first sheet)**

This international search report has not been established in respect of certain claims under Article 17(2)(a) for the following reasons:

1. ☐   Claims Nos.:
       because they relate to subject matter not required to be searched by this Authority, namely:

2. ☑   Claims Nos.: **10,11**
       because they relate to parts of the international application that do not comply with the prescribed requirements to such an extent that no meaningful international search can be carried out, specifically:

       Claims 10 and 11 refer to claims violating the manner of referring to dependent claims (PCT Rule 6.4(a)), and thus are unclear (PCT Article 6).

3. ☑   Claims Nos.: **8,9**
       because they are dependent claims and are not drafted in accordance with the second and third sentences of Rule 6.4(a).

Form PCT/ISA/210 (continuation of first sheet) (July 2019)

**INTERNATIONAL SEARCH REPORT**
Information on patent family members

International application No.

**PCT/KR2021/014991**

| Patent document cited in search report | Publication date (day/month/year) | Patent family member(s) | | Publication date (day/month/year) |
|---|---|---|---|---|
| KR 10-2016-0016756 A | 15 February 2016 | AU | 2013-203062 A1 | 02 October 2014 |
| | | AU | 2013-203062 B2 | 23 June 2016 |
| | | AU | 2016-231547 A1 | 06 October 2016 |
| | | AU | 2016-231547 B2 | 15 March 2018 |
| | | AU | 2018-203665 A1 | 14 June 2018 |
| | | AU | 2018-203665 B2 | 30 January 2020 |
| | | AU | 2020-202738 A1 | 14 May 2020 |
| | | BR | 112015023542 A2 | 18 July 2017 |
| | | BR | 112015023542 A8 | 03 December 2019 |
| | | CA | 2905058 A1 | 25 September 2014 |
| | | CN | 105407912 A | 16 March 2016 |
| | | EA | 034546 B1 | 19 February 2020 |
| | | EA | 201591758 A1 | 31 March 2016 |
| | | EA | 201591758 A8 | 31 March 2017 |
| | | EA | 201992832 A1 | 31 May 2020 |
| | | EP | 2968483 A1 | 20 January 2016 |
| | | EP | 2968483 B1 | 01 December 2021 |
| | | HK | 1220131 A1 | 28 April 2017 |
| | | IL | 241305 A | 30 November 2015 |
| | | IL | 241305 B | 30 April 2020 |
| | | IL | 241305 D0 | 30 November 2015 |
| | | IL | 273365 A | 31 May 2020 |
| | | IL | 273365 D0 | 31 May 2020 |
| | | JP | 2016-517421 A | 16 June 2016 |
| | | JP | 2019-142850 A | 29 August 2019 |
| | | JP | 6484216 B2 | 13 March 2019 |
| | | KR | 10-2019-0022926 A | 06 March 2019 |
| | | KR | 10-2020-0041394 A | 21 April 2020 |
| | | KR | 10-2021-0021107 A | 24 February 2021 |
| | | KR | 10-2102087 B1 | 20 April 2020 |
| | | KR | 10-2218489 B1 | 22 February 2021 |
| | | MX | 2015012783 A | 28 April 2016 |
| | | NZ | 712440 A | 27 November 2020 |
| | | NZ | 749570 A | 27 November 2020 |
| | | NZ | 751610 A | 27 November 2020 |
| | | SG | 10201707074 A | 30 October 2017 |
| | | SG | 10202001227 A | 30 March 2020 |
| | | SG | 11201507271 A | 29 October 2015 |
| | | TW | 201513881 A | 16 April 2015 |
| | | TW | 202017591 A | 16 May 2020 |
| | | TW | I690326 B | 11 April 2020 |
| | | TW | I740207 B | 21 September 2021 |
| | | US | 10413600 B2 | 17 September 2019 |
| | | US | 1185575 B2 | 30 November 2021 |
| | | US | 2014-0271611 A1 | 18 September 2014 |
| | | US | 2017-0224785 A1 | 10 August 2017 |
| | | US | 2020-0101144 A1 | 02 April 2020 |
| | | US | 9611467 B2 | 04 April 2017 |
| | | WO | 2014-151968 A1 | 25 September 2014 |
| | | WO | 2014-151968 A8 | 25 September 2014 |

Form PCT/ISA/210 (patent family annex) (July 2019)

**INTERNATIONAL SEARCH REPORT**
Information on patent family members

International application No.

**PCT/KR2021/014991**

| Patent document cited in search report | | | Publication date (day/month/year) | Patent family member(s) | | | Publication date (day/month/year) |
|---|---|---|---|---|---|---|---|
| KR | 10-1941514 | B1 | 23 January 2019 | AU | 2011-349049 | A1 | 28 June 2012 |
| | | | | AU | 2011-349049 | A1 | 02 May 2013 |
| | | | | AU | 2011-349049 | B2 | 11 August 2016 |
| | | | | BR | 112013016235 | A2 | 22 November 2016 |
| | | | | BR | 112013016235 | B1 | 31 March 2020 |
| | | | | CA | 2824279 | A1 | 28 June 2012 |
| | | | | CN | 103429261 | A | 04 December 2013 |
| | | | | EA | 201390923 | A1 | 30 December 2013 |
| | | | | EP | 2654790 | A1 | 30 October 2013 |
| | | | | EP | 2654790 | B1 | 06 February 2019 |
| | | | | ES | 2720136 | T3 | 18 July 2019 |
| | | | | IL | 227081 | A | 29 August 2013 |
| | | | | IL | 227081 | B | 30 April 2018 |
| | | | | JP | 2014-503209 | A | 13 February 2014 |
| | | | | JP | 2017-055771 | A | 23 March 2017 |
| | | | | JP | 6147670 | B2 | 14 June 2017 |
| | | | | JP | 6334669 | B2 | 30 May 2018 |
| | | | | MX | 2013007392 | A | 01 November 2013 |
| | | | | PT | 2654790 | T | 16 May 2019 |
| | | | | US | 2013-0281677 | A1 | 24 October 2013 |
| | | | | US | 9505826 | B2 | 29 November 2016 |
| | | | | WO | 2012-083370 | A1 | 28 June 2012 |
| | | | | ZA | 201305123 | B | 25 September 2014 |
| US | 2015-0044171 | A1 | 12 February 2015 | US | 9546360 | B2 | 17 January 2017 |
| | | | | WO | 2013-096793 | A1 | 27 June 2013 |
| US | 2019-0161746 | A1 | 30 May 2019 | AT | 544866 | T | 15 February 2012 |
| | | | | AU | 2006-257073 | A1 | 21 December 2006 |
| | | | | AU | 2006-257073 | B2 | 04 August 2011 |
| | | | | CA | 2606351 | A1 | 21 December 2006 |
| | | | | CA | 2606351 | C | 13 December 2016 |
| | | | | DK | 1902141 | T3 | 07 May 2012 |
| | | | | EP | 1902141 | A1 | 26 March 2008 |
| | | | | EP | 1902141 | B1 | 08 February 2012 |
| | | | | ES | 2382104 | T3 | 05 June 2012 |
| | | | | HK | 1118579 | A1 | 13 February 2009 |
| | | | | JP | 2009-539757 | A | 19 November 2009 |
| | | | | PL | 1902141 | T3 | 31 December 2012 |
| | | | | US | 10233436 | B2 | 19 March 2019 |
| | | | | US | 1124787 | B2 | 21 September 2021 |
| | | | | US | 2007-0015703 | A1 | 18 January 2007 |
| | | | | US | 2013-0136732 | A1 | 30 May 2013 |
| | | | | WO | 2006-133955 | A1 | 21 December 2006 |
| | | | | WO | 2006-133955 | A8 | 29 November 2007 |
| WO | 2017-055908 | A1 | 06 April 2017 | EP | 3356402 | A1 | 08 August 2018 |
| | | | | JP | 2018-528975 | A | 04 October 2018 |
| | | | | US | 2018-0237509 | A1 | 23 August 2018 |

Form PCT/ISA/210 (patent family annex) (July 2019)

# EP 4 249 594 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **HUANG et al.** *Comp. Appl. BioSci.,* 1992, vol. 8, 155-65
- **PEARSON et al.** *Meth. Mol. Biol.,* 1994, vol. 24, 307-31
- **ALTSCHUL et al.** *J. Mol. Biol.,* 1990, vol. 215, 403-10
- *Remington's Pharmaceutical Sciences,* 1995
- **MIESBACH W ; MENNE J ; BOMMER M ; SCHONERMARCK U ; FELDKAMP T ; NITSCHKE M ; WESTHOFF TH ; SEIBERT FS ; WOITAS R ; SOUSA R.** *Orphanet Journal of Rare Diseases,* 2019, vol. 14, 260
- **ERCIG B ; WICHAPONG K ; REUTELINGSPERGER CPM ; VANHOORELBEKE K ; VOORBERG J ; NICOLAES GAF.** *Thromb Haemost.,* 2018, vol. 118 (1), 28-41
- **JOHANNA A KREMER HOVING ; PAUL COPPO ; BERNHARD LAMMLE ; JOEL L MOAKE ; TOSHIYUKI MIYATA ; KAREN VANHOORELBEKE.** *Nat Rev Dis Primers,* 06 April 2017, vol. 3, 17020
- *Genetic and Rare Diseases Information Center (GARD)-an NCATS Program,* 10 October 2018
- **TERSTEEG C ; VERHENNE S ; ROOSE E et al.** *Expert Rev Hematol.,* 2016, vol. 9 (2), 209-221
- **KLAUS C ; PLAIMAUER B ; STUDT JD et al.** *Blood.,* 2004, vol. 103 (12), 4514-4519
- **LUKEN BM ; TURENHOUT EA ; HULSTEIN JJ ; VAN MOURIK JA ; FIJNHEER R ; VOORBERG J.** *Thromb Haemost.,* 2005, vol. 93 (2), 267-274
- **VERONICA C. CASINA ; WENBING HU ; JIAN-HUA MAO ; RUI-NAN LU ; HAYLEY A. HANBY ; BRANDY PICKENS ; ZHONG-YUAN KAN ; WOON K. LIM ; LELAND MAYNE ; ERIC M. OSTERTAG.** *Proc Natl Acad Sci USA.,* 04 August 2015, vol. 112 (31), 9620-5
- **SURESH G SHELAT ; JIHUI AI ; X. LONG ZHENG.** *Semin Thromb Hemost.,* December 2005, vol. 31 (6), 659-72